# EUROPÄISCHE PATENTSCHRIFT

(11) **EP 3 067 011 B1**
(45) Veröffentlichungstag und Bekanntmachung des Hinweises auf die Patenterteilung: **25.05.2022**
(21) Anmeldenummer: 16159490.8
(22) Anmeldetag: 09.03.2016
(51) Int. Cl.: H04N 13/20, A61B 1/24, H04N 13/296, H04N 13/239, A61C 9/00, A61B 1/00

(54) **DENTALBILDERFASSUNGSVORRICHTUNG**
DENTAL IMAGING DEVICE
DISPOSITIF DE FORMATION D'IMAGES DENTAIRES

(30) Priorität: 09.03.2015 DE 102015103416; 09.03.2015 DE 102015103413
(43) Veröffentlichungstag der Anmeldung: 14.09.2016
(73) Patentinhaber: Renfert GmbH, 78247 Hilzingen (DE)
(72) Erfinder: Egelhof, Joachim, 73655 Plüderhausen (DE)
(74) Vertreter: Daub, Thomas

(56) Entgegenhaltungen:
- US-A1- 2005 088 529
- US-A1- 2005 089 822
- US-A1- 2013 258 139
- US-A1- 2015 042 761

## Beschreibung

### Stand der Technik

Die Erfindung betrifft eine Dentalbilderfassungsvorrichtung.

Es ist bereits eine Dentalbilderfassungsvorrichtung, insbesondere eine Dentaltechnikbilderfassungsvorrichtung, vorgeschlagen worden, mit einer Kameraeinheit zur Aufnahme stereoskopischer Bilder, die zumindest zwei Kameramodule aufweist, und mit zumindest einer Steuer- und/oder Regeleinheit, die Signale der Kameraeinheit verarbeitet und zumindest ein Ausgabesignal liefert, das dazu vorgesehen ist, einer Anzeigeeinheit zugeführt zu werden.

Aus der US 2005/0089822 A1 ist bereits eine Dentalbilderfassungsvorrichtung mit einer Kameraeinheit zur Aufnahme stereoskopischer Bilder bekannt. Ferner ist aus der US 2015/0042761 A1 bereits ein Verfahren und eine Vorrichtung zur Bildhelligkeitssteuerung in einer Stereokamera bekannt.

Die Aufgabe der Erfindung besteht insbesondere darin, eine gattungsgemäße Vorrichtung mit verbesserten Eigenschaften hinsichtlich eines Komforts sowie hinsichtlich einer Variabilität bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung wird durch den unabhängigen Anspruch definiert.

Die Erfindung geht aus von einer Dentalbilderfassungsvorrichtung, insbesondere vor einer Dentalbilderfassungsvorrichtung, mit einer Kameraeinheit zur Aufnahme stereoskopischer Bilder, die zumindest zwei Kameramodule aufweist, und mit zumindest einer Steuer- und/oder Regeleinheit, die Signale der Kameraeinheit verarbeitet und zumindest ein Ausgabesignal liefert, das dazu vorgesehen ist, einer Anzeigeeinheit zugeführt zu werden.

Es wird vorgeschlagen, dass die zumindest eine Steuer- und/oder Regeleinheit zumindest ein Speicherelement aufweist, auf welchem zumindest ein Datenprofil hinterlegt ist. Vorzugsweise sind auf dem Speicherelement mehrere differierende Datenprofile hinterlegt.

Unter einer "Dentalbilderfassungsvorrichtung" soll insbesondere eine Vorrichtung verstanden werden, die dazu vorgesehen ist, von Dentaltechnikern und/oder Ärzten, insbesondere Zahnärzten, an einem Zahnarztstuhl und/oder bei der Bearbeitung von zumindest einem zu bearbeitenden Werkstück, also Zahnersatz und/oder Zahnersatzvorstufen, insbesondere Gebissen, Brücken, Passungen und/oder Zähnen, eingesetzt zu werden, um ein Bild eines Mundraums eines Patienten auf dem Zahnarztstuhl und/oder des zu bearbeitenden Werkstücks aufzunehmen. Unter einer "Dentaltechnikbilderfassungsvorrichtung" soll insbesondere eine Vorrichtung verstanden werden, die dazu vorgesehen ist, von Dentaltechnikern und/oder Ärzten, insbesondere Zahnärzten, bei der Bearbeitung, insbesondere Ausformung, Modellierung, spanenden Bearbeitung, Bemalung und/oder Einfärbung, von zumindest einem zu bearbeitenden Werkstück, also Zahnersatz und/oder Zahnersatzvorstufen, insbesondere Gebissen, Brücken, Passungen und/oder Zähnen, eingesetzt zu werden, um ein Bild des zu bearbeitenden Werkstücks aufzunehmen und hohe Genauigkeiten bei Randschlüssen und/oder Präparationsgrenzen zu erreichen. Insbesondere ist es denkbar, dass ein von einer Dentaltechnikbilderfassungsvorrichtung erzeugtes Ausgangssignal, das von/bzw. bei einem Dentaltechniker aufgenommen wurde, insbesondere über ein Netzwerk, vorteilhaft das Internet, an eine Anzeigeeinheit eines Zahnarztes übermittelt wird bzw. umgekehrt.

Unter einem "Kameramodul" soll insbesondere ein Modul verstanden werden, das zumindest einen, vorzugsweise elektrischen, optischen Flächensensor, insbesondere einen CCD-Sensor, und zumindest eine Optikeinheit aufweist, die dazu vorgesehen ist, zumindest einen Objektbereich auf dem CCD-Sensor abzubilden. Alternativ sind Ausgestaltungen der Kameramodule als Zeilensensor denkbar, wobei durch eine Bewegung des zu beobachtenden Objekts oder durch eine Bewegung, insbesondere eine Schwenkbewegung, der Kameramodule, ein höherdimensionales Bild generierbar ist. Insbesondere sind die Kameramodule als Web-Cam-Module ausgebildet, wodurch eine preiswerte Ausgestaltung erreicht werden kann. Insbesondere sind die Kameramodule nebeneinander angeordnet, wobei optische Achsen der Kameramodule vorzugsweise ein gleichschenkliges Dreieck bilden und insbesondere einen Winkel von maximal 30°, insbesondere maximal 20° und vorteilhaft maximal 15° und insbesondere von mindestens 5° und vorteilhaft von mindestens 10° einschließen. Alternativ sind Ausgestaltungen denkbar, wobei optische Achsen der Kameramodule im Wesentlichen senkrecht zueinander angeordnet sind und über zumindest eine Prisma- und/oder Spiegelanordnung, insbesondere zumindest einem halbdurchlässigen Spiegel, umgelenkt sind.

Unter einer "Steuer- und/oder Regeleinheit" soll insbesondere eine Einheit verstanden werden, die zumindest eine Recheneinheit, zumindest ein Speicherelement und zumindest ein in dem Speicherelement hinterlegtes Betriebsprogramm aufweist, das dazu vorgesehen ist, von der Steuer- und/oder Regeleinheit ausgeführt zu werden. Insbesondere ist die Steuer- und/oder Regeleinheit dazu vorgesehen, die Signale der Kameraeinheit, die vorzugsweise direkten Signalen der Kameramodule entsprechen, zu verarbeiten und zu einem Ausgabesignal zusammenzufügen, das dazu geeignet ist, von zumindest einer stereoskopischen Anzeigeeinheit, insbesondere von beliebigen stereoskopischen Anzeigeeinheiten, als stereoskopisches Bild ausgegeben zu werden. Insbesondere ist die Steuer- und/oder Regeleinheit dazu vorgesehen, die Kameraeinheit, bzw. die Kameramodule mit einem Synchronisierungssignal, insbesondere einem V-Sync-Signal, anzusprechen, um von beiden Kameramodulen gleichzeitig Bilddaten abzufragen. Alternativ ist es denkbar, dass die Steuer- und/oder Regeleinheit dazu vorgesehen ist, Bilddaten der Kameramodule abwechselnd abzufragen, um für Shutterbrillen optimierte Bilddaten zu erhalten.

Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt. Darunter, dass "das Ausgabesignal dazu vorgesehen ist, einer Anzeigeeinheit zugeführt" zu werden, soll insbesondere verstanden werden, dass die Dentalbilderfassungsvorrichtung zumindest eine Schnittstelle, insbesondere eine Grafikschnittstelle, beispielsweise eine HDMI-Schnittstelle, VGA-Schnittstelle oder Vergleichbares, alternativ eine drahtlose und/oder drahtgebundene Datenschnittstelle, insbesondere eine Netzwerkschnittstelle, eine USB-Schnittstelle oder Vergleichbares, aufweist, die dazu vorgesehen ist, mit der Anzeigeeinheit verbunden zu werden. Insbesondere weist die Dentaltechnikvorrichtung zumindest eine Halteeinheit auf, die dazu vorgesehen ist, einen Tablet-PC und/oder ein Smartphone aufzunehmen, der bzw. das dazu vorgesehen ist, das Ausgabesignal zu empfangen und in eine stereoskopische Anzeige zu wandeln.

Unter einem "Speicherelement" soll insbesondere ein Element und/oder ein Mittel verstanden werden, das dazu vorgesehen ist, zumindest eine Information, insbesondere Daten, vorteilhaft von einer Stromversorgung unabhängig, zu speichern. Erfindungsgemäß soll in diesem Zusammenhang unter einem "Datenprofil" insbesondere ein Datensatz, bevorzugt ein digitaler Datensatz, verstanden werden, welcher ein oder mehrere Einstellungen und/oder Konfigurationen umfasst. Vorzugsweise soll darunter insbesondere ein Datensatz verstanden werden, welcher zumindest eine Zuordnung aufweist. Bevorzugt ist der Datensatz beispielsweise einem Material und/oder einem Benutzer und/oder einer Anwendung und/oder einem Patienten zugeordnet. Grundsätzlich sind jedoch auch andere, einem Fachmann als sinnvoll erscheinende Zuordnungen denkbar. Die Datenprofile sind daher insbesondere in Untergruppen unterteilt. Beispielsweise kann zwischen Materialdatenprofilen, Anwendungsdatenprofilen, Patientendatenprofilen und/oder Nutzerdatenprofilen unterschieden werden. Materialdatenprofile umfassen vorzugsweise mehrere Einstellwerte, insbesondere mehrere Bildbearbeitungsparameter und/oder zumindest eine Funktion und/oder eine Zuordnungstabelle, insbesondere Lookup-Tabelle, einer Gradationskurve. Bei einer Anwendung an einem Zahnarztstuhl wären beispielsweise verschiedene Patientendatenprofilen für verschiedene Gebissfärbungen, wie beispielsweise für ein Rauchergebiss, denkbar. Nutzerdatenprofile werden vorzugsweise zumindest teilweise manuell von einem Nutzer und/oder Bediener eingestellt. Besonders bevorzugt sind die Nutzerdatenprofile jeweils einem definierten Nutzer und/oder Bediener zugeordnet. Unter einem "Material" soll erfindungsgemäß insbesondere ein Material eines zu beobachtenden Objekts verstanden werden. Es sind verschiedene, einem Fachmann als sinnvoll erscheinende Materialen, wie beispielsweise Gold und/oder Zirkonoxid, denkbar.

Unter einer "Gradationskurve" soll dabei insbesondere eine Zuordnungstabelle und/oder eine Kurve, welche insbesondere durch eine Funktion definiert ist, verstanden werden, welche ein Verhältnis zwischen Ein- und Ausgabe von Tonwerten definiert. Vorzugsweise beschreibt die Kurve ein Verhältnis ursprünglicher Tonwerte zu neuen Tonwerten. Bevorzugt sind dabei auf einer ersten Achse, insbesondere x-Achse, die ursprünglichen Eingangstonwerte und auf einer zweiten Achse, insbesondere y-Achse, die neuen Ausgangstonwerte aufgetragen. Die Tonwerte erstrecken sich dabei insbesondere von schwarz nach weiß. Die Gradationskurve kann dabei insbesondere als Lookup-Tabelle ausgeführt sein. Hierdurch kann ein Rechenaufwand vorteilhaft gering gehalten werden. Unter einem "Tonwert" soll in diesem Zusammenhang insbesondere ein Farbwert zwischen Hell und Dunkel eines Farb- oder Schwarzweiß-Bildes verstanden werden. Vorzugsweise soll darunter ein Wert verstanden werden, welcher bei einem Bildelement, insbesondere einem Pixel, einen Farb- oder Grauwert innerhalb eines vorgegebenen Farb- bzw. Graustufenspektrums beschreibt. Vorzugsweise soll darunter ein Wert verstanden werden, welcher unabhängig von einer Farbe, eine Helligkeit eines Bildelements, insbesondere eines Pixels, beschreibt.

Durch die erfindungsgemäße Ausgestaltung der Dentalbilderfassungsvorrichtung kann insbesondere eine schnelle Zuordnung von Einstellungen und/oder Konfigurationen erreicht werden. Insbesondere kann so eine komfortable Zuordnung erreicht werden. Vorzugsweise kann so abhängig von einer aktuellen Situation, einem aktuell verwendeten Material und/oder einem aktuellen Benutzer ein Datenprofil ausgewählt bzw. an- und/oder abgewählt werden. So kann insbesondere eine schnelle Einstellung erreicht werden.

Insbesondere ist die Steuer- und/oder Regeleinheit dazu vorgesehen, insbesondere zumindest abhängig von einer Bedienereingabe, einen Teilausschnitt eines beobachteten Objektbereichs, vorzugsweise digital vergrößert, in das Ausganssignal einzubetten. Vorteilhaft weist die Dentalbilderfassungsvorrichtung zumindest ein Stativ mit insbesondere zumindest einem Schwenkarm auf, wobei das Stativ an einem oberen Ende zumindest eine Halteeinheit für die Kameramodule aufweist. Insbesondere weist das Stativ an einem unteren Ende zumindest eine Montageeinheit auf, die dazu vorgesehen ist, das Stativ, vorzugsweise über eine Schraub- und/oder Klemmverbindung, mit einem Arbeitsplatz, insbesondere einer Arbeitsplatte, zu verbinden. Insbesondere weist die Dentalbilderfassungsvorrichtung zumindest eine Beleuchtungseinheit auf, die dazu vorgesehen ist, zumindest einen Objektbereich auszuleuchten. Vorzugsweise ist die Beleuchtungseinheit von zumindest einer LED, insbesondere zumindest einer Gruppe von LED's, gebildet. Alternativ ist es denkbar, dass die Beleuchtungseinheit dazu vorgesehen ist, ein zu beobachtendes Objekt über zumindest einen halbdurchlässigen Spiegel, der vorteilhaft in zumindest einer der optischen Achsen der Kameramodule liegt, zu beleuchten. Vorteilhaft weist die Dentalbilderfassungsvorrichtung zumindest eine Eingabeeinheit auf, die dazu vorgesehen ist, einem Bediener eine Auswahl von Funktionen und/oder Betriebsmodi zu ermöglichen. Alternativ oder zusätzlich ist es denkbar, dass die Steuer- und/oder Regeleinheit dazu vorgesehen ist, mit zumindest einem externen Eingabegerät, insbesondere einer in einem angeschlossenen Monitor integrierten Touchoberfläche, einem über eine Datenverbindung gekoppelten Smartphone, einer, beispielsweise über USB angeschlossenen, Maus und/oder Tastatur, gekoppelt zu werden, wobei die Steuer- und/oder Regeleinheit insbesondere eine Bedienoberfläche implementiert und über das Ausgabesignal ausgibt. Insbesondere ist die Steuer- und/oder Regeleinheit dazu vorgesehen, in zumindest einem Betriebsmodus ein aufgenommenes reales Modell in ein rechnergestütztes Modell zur Verwendung in einem CAD-CAM-Verfahren umzuwandeln. Insbesondere ist es denkbar, dass zumindest die Kameraeinheit in einer Fräsmachine, insbesondere zur Überwachung eines Arbeitsablaufs und/oder eines Arbeitsergebnisses, angeordnet ist. Weiterhin ist es denkbar, dass die Steuer- und/oder Regeleinheit dazu vorgesehen ist, in Abhängigkeit von einer Bedienereingabe zwischen einem stereoskopischen Ausgangssignal und einem klassischen Ausgangssignal umzuschalten.

Ferner wird vorgeschlagen, dass auf dem zumindest einen Speicherelement der zumindest einen Steuer- und/oder Regeleinheit mehrere zu einer Auswahl stehende Datenprofile hinterlegt sind. Dadurch kann insbesondere eine schnelle Zuordnung von Einstellungen und/oder Konfigurationen erreicht werden. Insbesondere kann so eine komfortable Zuordnung erreicht werden. Vorzugsweise kann so abhängig von einer aktuellen Situation, einem aktuell verwendeten Material und/oder einem aktuellen Benutzer ein Datenprofil ausgewählt werden. So kann insbesondere eine schnelle Einstellung erreicht werden.

Erfindungsgemäß wird vorgeschlagen, dass auf dem zumindest einen Speicherelement zumindest ein Datenprofil hinterlegt ist, welches einem Material zugeordnet ist. Erfindungsgemäß sind auf dem zumindest einen Speicherelement mehrere Datenprofile hinterlegt, die jeweils einem Material zugeordnet sind. Erfindungsgemäß sind die Datenprofile dabei auf eine vorteilhafte Ausleuchtung des Materials bei gleichzeitiger Vermeidung von Reflektionen ausgelegt. Auf dem zumindest einen Speicherelement können insbesondere verschiedene Datenprofile mit differierenden Zuordnungen hinterlegt sein. Es sind verschiedene, einem Fachmann als sinnvoll erscheinende Zuordnungen denkbar. Dadurch kann insbesondere eine schnelle Zuordnung von Einstellungen und/oder Konfigurationen erreicht werden. Vorzugsweise kann so abhängig von einem aktuell verwendeten Material ein Datenprofil ausgewählt werden. So kann insbesondere eine schnelle Einstellung eines aktuell verwendeten Materials erreicht werden.

Erfindungsgemäß wird vorgeschlagen, dass die zumindest eine Steuer- und/oder Regeleinheit dazu vorgesehen ist, abhängig von einem ausgewählten, auf dem Speicherelement hinterlegten Datenprofil die Signale der Kameraeinheit und/oder ein Ausgabesignal anzupassen. Vorzugsweise ist die zumindest eine Steuer- und/oder Regeleinheit dazu vorgesehen, abhängig von einem ausgewählten, auf dem Speicherelement hinterlegten Datenprofil ein digitales Bildsignal der Kameraeinheit vor einer Erzeugung eines Ausgabesignals zu bearbeiten. Vorzugsweise ist die Steuer- und/oder Regeleinheit dazu vorgesehen, abhängig von einem ausgewählten, auf dem Speicherelement hinterlegten Datenprofil ein digitales Bildsignal der Kameraeinheit vor einer Erzeugung eines Ausgabesignals mittels digitaler Bildbearbeitung zu bearbeiten. Bevorzugt ist die zumindest eine Steuer- und/oder Regeleinheit dazu vorgesehen, zur Vermeidung von Reflektionen, abhängig von einem ausgewählten, auf dem Speicherelement hinterlegten Datenprofil, ein digitales Bildsignal der Kameraeinheit vor einer Erzeugung eines Ausgabesignals zu bearbeiten. Besonders bevorzugt wird ein digitales Bildsignal der Kameraeinheit vor einer Erzeugung eines Ausgabesignals aufgehellt, wobei dunkle Pixel um einen deutlich höheren Faktor angehoben werden als helle Pixel. Vorzugsweise wird, um die Übersteuerung an Glanzstellen zu minimieren, die Belichtungszeit stark reduziert. Hierdurch stehen die Glanzstellen insbesondere kurz vor der Übersteuerung, der Rest des Bildes ist jedoch stark abgedunkelt. Um die nun zu dunklen Stellen wieder anzuheben, wird vorzugsweise eine Gradationskurve auf ein digitales Bildsignal der Kameraeinheit angewendet. Hierbei werden insbesondere dunkle Pixel deutlich stärker angehoben als bereits helle Pixel. Ferner wird ein Bild besonders bevorzugt bereits relativ dunkel aufgenommen. Dadurch kann insbesondere eine gezielte Anpassung von Signalen erreicht werden. Vorzugsweise kann ein Ausgabesignal vorteilhaft an ein aktuell ausgewähltes Datenprofil angepasst werden.

In der Regel kann es bei glänzenden Materialien immer wieder zu größeren Reflektionen kommen. Diese größeren Reflektionen beeinträchtigen eine stereoskopische Bildwiedergabe, sodass in diesen Bereichen für einen Bediener keine Tiefeninformation wahrgenommen werden kann. Dies kann dadurch zumindest teilweise behoben werden. So kann vorteilhaft eine Ausgabequalität verbessert werden.

Zudem oder alternativ wäre denkbar, dass die zumindest eine Steuer- und/oder Regeleinheit dazu vorgesehen ist, abhängig von einem ausgewählten, auf dem Speicherelement hinterlegten Datenprofil eine Beleuchtungseinheit der Dentalbilderfassungsvorrichtung anzupassen. Dabei können insbesondere einzelne Beleuchtungselemente gezielt gedimmt oder deaktiviert werden. Dadurch kann insbesondere gezielt die Beleuchtungseinheit der Dentalbilderfassungsvorrichtung angepasst werden. Vorzugsweise kann die Beleuchtungseinheit der Dentalbilderfassungsvorrichtung an eine aktuelle Situation angepasst werden. Zudem oder alternativ wäre auch denkbar, dass die zumindest eine Steuer- und/oder Regeleinheit dazu vorgesehen ist, abhängig von einem ausgewählten, auf dem Speicherelement hinterlegten Datenprofil, die eine Lage der Kameraeinheit anzupassen. So könnte insbesondere eine besonders vorteilhafte Anpassung erfolgen. Bei einer Anwendung an einem Zanarztstuhl wäre beispielsweise denkbar, dass über ein Datenprofil eine indirekte Blendwirkung für einen Patienten und/oder einen Bediener gesteuert und/oder geregelt werden kann. Es kann beispielsweise eine Leuchtfläche in seiner Form und Größe angepasst werden.

Es wird weiter vorgeschlagen, dass die Dentalbilderfassungsvorrichtung zumindest eine Eingabeeinheit aufweist, welche zu einer Auswahl eines auf dem zumindest einen Speicherelement der zumindest einen Steuer- und/oder Regeleinheit hinterlegten Datenprofils vorgesehen ist. Vorzugsweise kann über die Eingabeeinheit manuell ein Datenprofil ausgewählt werden. Bevorzugt kann ein Bediener über die Eingabeeinheit ein Datenprofil auswählen. Grundsätzlich wäre jedoch auch denkbar, dass über die Eingabeeinheit ein Datenprofil angelegt werden kann und/oder Parameter eines Datenprofils bearbeitet werden können. Unter einer "Eingabeeinheit" soll hier insbesondere eine Einheit verstanden werden, die zumindest ein Bauteil aufweist, das direkt von einem Bediener betätigbar ist, und die dazu vorgesehen ist, durch eine Betätigung und/oder durch eine Eingabe von Parametern einen Prozess und/oder einen Zustand einer mit der Eingabeeinheit gekoppelten Einheit zu beeinflussen und/oder zu ändern. Unter einer "Auswahl" kann dabei sowohl das An- und/oder Abwählen eines einzelnen Datenprofils, als auch die Auswahl eines Datenprofils aus mehreren Datenprofilen verstanden werden. Es ist daher sowohl denkbar, dass auf dem Speicherelement lediglich ein Datenprofil hinterlegt ist, als auch bevorzugt, dass auf dem Speicherelement mehrere Datenprofile zu einer Auswahl hinterlegt sind. Dadurch kann insbesondere eine manuelle Auswahl eines Datenprofils erreicht werden. So kann insbesondere eine schnelle Einstellung von Einstellungen und/oder Konfigurationen erreicht werden.

Erfindungsgemäß wird vorgeschlagen, dass die Dentalbilderfassungsvorrichtung zumindest eine Sensoreinheit aufweist, welche mit der zumindest einen Steuer- und/oder Regeleinheit verbunden ist. Vorzugsweise ist die Sensoreinheit dazu vorgesehen, ein verwendetes Material zu erfassen. Bevorzugt ist die Sensoreinheit dazu vorgesehen, optisch ein verwendetes Material zu erkennen. Unter einer "Sensoreinheit" soll in diesem Zusammenhang insbesondere eine Einheit verstanden werden, die dazu vorgesehen ist, zumindest eine Kenngröße und/oder eine physikalische Eigenschaft aufzunehmen, wobei die Aufnahme aktiv, wie insbesondere durch Erzeugen und Aussenden eines elektrischen Messsignals, und/oder passiv, wie insbesondere durch eine Erfassung von Eigenschaftsänderungen eines Sensorbauteils, stattfinden kann. Es sind verschiedene, dem Fachmann als sinnvoll erscheinende Sensoreinheiten denkbar. Dadurch kann insbesondere eine vorteilhafte Dentalbilderfassungsvorrichtung bereitgestellt werden. Insbesondere kann eine Überwachung von Kenngrößen erreicht werden.

Ferner wird erfindungsgemäß vorgeschlagen, dass die zumindest eine Steuer- und/oder Regeleinheit dazu vorgesehen ist, abhängig von einem Signal der zumindest einen Sensoreinheit, selbsttätig ein auf dem zumindest einen Speicherelement der zumindest einen Steuer- und/oder Regeleinheit hinterlegtes Datenprofil auszuwählen. Dadurch kann insbesondere eine selbsttätige Auswahl eines Datenprofils erreicht werden. So kann insbesondere eine schnelle und komfortable Einstellung von Einstellungen und/oder Konfigurationen erreicht werden. Es können falsche Einstellungen vermieden werden.

Es wird weiter vorgeschlagen, dass die Dentalbilderfassungsvorrichtung zumindest eine Schnittstelle aufweist, über welche ein externes Datenprofil auf das zumindest eine Speicherelement der zumindest einen Steuer- und/oder Regeleinheit übertragen werden kann. Vorzugsweist ist die Schnittstelle als eine Datenschnittstelle ausgebildet. Bevorzugt können über die Schnittstelle zusätzliche, beispielsweise selbst erstellte Datenprofile auf das zumindest eine Speicherelement der zumindest einen Steuer- und/oder Regeleinheit übertragen werden. Bevorzugt können über die zumindest eine Schnittstelle auch Datenprofile von dem zumindest einen Speicherelement der zumindest einen Steuer- und/oder Regeleinheit herunterkopiert werden. Unter einer "Schnittstelle" soll in diesem Zusammenhang insbesondere eine Einheit verstanden werden, welche zu einem Austausch von Daten vorgesehen ist. Insbesondere weist die Schnittstelle zumindest einen Informationseingang und zumindest einen Informationsausgang auf. Vorzugsweise weist die Schnittstelle zumindest zwei Informationseingänge und zumindest zwei Informationsausgänge auf, wobei jeweils zumindest ein Informationseingang und zumindest ein Informationsausgang zu einer Verbindung mit einem physischen System vorgesehen sind. Besonders bevorzugt soll darunter eine Schnittstelle zwischen zumindest zwei physischen Systemen, wie insbesondere zwischen der Dentalbilderfassungsvorrichtung und zumindest einem oder mehreren Geräten der Außenwelt, verstanden werden. Es sind verschiedene, dem Fachmann als sinnvoll erscheinende Schnittstellen denkbar, insbesondere soll darunter jedoch eine drahtlose Schnittstelle, wie beispielsweise Bluetooth, WLAN oder NFC, eine drahtgebundene Schnittstelle, wie beispielsweise ein USB-Anschluss, und/oder eine Laufwerk-Schnittstelle mittels eines Speichermediums, wie beispielsweise einer Speicherkarte, eines Speichersticks oder einer CD, verstanden werden. Dadurch können vorteilhaft zusätzliche Datenprofile auf die Dentalbilderfassungsvorrichtung aufgespielt werden. Vorzugsweise auch externe Datenprofile auf die Dentalbilderfassungsvorrichtung aufgespielt werden. Ferner können Datenprofile so auch extern erstellt oder bearbeitet werden.

Es wird ferner vorgeschlagen, dass die zumindest eine Steuer- und/oder Regeleinheit dazu vorgesehen ist, eine Momentaufnahme mit einem aktuell ausgewählten Datenprofil zu verknüpfen. Vorzugsweise bietet die Dentalbildererfassungsvorrichtung die Möglichkeit, Momentaufnahmen über die Schnittstelle oder eine weitere Schnittstelle an ein externes Gerät zu übertragen. So können Momentaufnahmen beispielsweise an einen Zahnarzt oder einen Zahntechniker übertragen werden. Durch die Verknüpfung mit dem aktuell ausgewählten Datenprofil kann insbesondere erreicht werden, dass ein Empfänger nachvollziehen kann, unter welchen Bedingungen die Momentaufnahme gemacht wurde. So kann beispielsweise auch ein Originalzustand der Momentaufnahme rekonstruiert werden. Unter einer "Momentaufnahme" soll in diesem Zusammenhang insbesondere eine Momentaufnahme eines Ausgangssignal verstanden werden. Vorzugsweise soll darunter eine Bildschirmkopie der Anzeigeeinheit verstanden werden. Bevorzugt soll darunter ein aktuell aufgenommenes Bild verstanden. Dadurch können insbesondere die Aufnahmebedingungen nachvollzogen werden. Vorzugsweise kann so vorteilhaft ein Originalzustand rekonstruiert werden.

Ferner wird vorgeschlagen, dass die zumindest eine Steuer- und/oder Regeleinheit dazu vorgesehen ist, abhängig von zumindest einem Parameter ein digitales Bildsignal der Kameraeinheit vor einer Erzeugung eines Ausgabesignals zu bearbeiten. Vorzugsweise ist die Steuer- und/oder Regeleinheit dazu vorgesehen, abhängig von zumindest einem Parameter ein digitales Bildsignal der Kameraeinheit vor einer Erzeugung eines Ausgabesignals mittels digitaler Bildbearbeitung zu bearbeiten. Bevorzugt ist die zumindest eine Steuer- und/oder Regeleinheit dazu vorgesehen, zur Vermeidung von Reflektionen abhängig von zumindest einem Parameter ein digitales Bildsignal der Kameraeinheit vor einer Erzeugung eines Ausgabesignals zu bearbeiten. Besonders bevorzugt wird ein digitales Bildsignal der Kameraeinheit vor einer Erzeugung eines Ausgabesignals aufgehellt, wobei dunkle Pixel um einen deutlich höheren Faktor angehoben werden als helle Pixel. Ferner wird ein Bild besonders bevorzugt bereits relativ dunkel aufgenommen. Darunter, dass "die Steuer- und/oder Regeleinheit dazu vorgesehen ist, ein digitales Bildsignal der Kameraeinheit zu bearbeiten" soll in diesem Zusammenhang insbesondere verstanden werden, dass die Steuer- und/oder Regeleinheit dazu vorgesehen ist, ein digitales Bildsignal, insbesondere mittels Bildbearbeitung, zu korrigieren. Vorzugsweise soll darunter insbesondere verstanden werden, dass die Steuer- und/oder Regeleinheit dazu vorgesehen ist, ein digitales Bildsignal mittels Bildbearbeitung, insbesondere mittels digitaler Bildbearbeitung, zu bearbeiten, insbesondere zu korrigieren. Darunter, dass die Bearbeitung "abhängig von zumindest einem Parameter" erfolgt, soll in diesem Zusammenhang insbesondere verstanden werden, dass eine Art und/oder ein Intensität einer Bearbeitung abhängig von zumindest einem Parameter erfolgt. Eine Bearbeitung variiert insbesondere abhängig von zumindest einem Parameter. Dadurch kann insbesondere eine vorteilhaft hohe Bildqualität erreicht werden. Insbesondere kann eine hohe Ausgabequalität erreicht werden. Ferner können Störeinflüsse vermieden bzw. korrigiert werden. Vorteilhaft können Reflektionen vermieden werden, insbesondere ohne eine Bildhelligkeit zu beeinträchtigen. In der Regel kann es bei glänzenden Materialien immer wieder zu größeren Reflektionen kommen. Diese größeren Reflektionen beeinträchtigen eine stereoskopische Bildwiedergabe, sodass in diesen Bereichen für einen Bediener keine Tiefeninformation wahrgenommen werden kann. Dies kann hierdurch zumindest teilweise behoben werden. So kann vorteilhaft eine Ausgabequalität verbessert werden.

In einer alternativen Ausgestaltung sind diese Merkmale der Erfindung, wonach die zumindest eine Steuer- und/oder Regeleinheit dazu vorgesehen ist, abhängig von zumindest einem Parameter ein digitales Bildsignal der Kameraeinheit vor einer Erzeugung eines Ausgabesignals zu bearbeiten, insbesondere in Kombination mit den Merkmalen, wonach die Dentalbilderfassungsvorrichtung eine Kameraeinheit, mit zumindest zwei Kameramodulen, zur Aufnahme stereoskopischer Bilder und zumindest eine Steuer- und/oder Regeleinheit aufweist, die Signale der Kameraeinheit verarbeitet und zumindest ein Ausgabesignal liefert, das dazu vorgesehen ist, einer Anzeigeeinheit zugeführt zu werden, auch unabhängig von den Merkmalen der Erfindung, wonach die zumindest eine Steuer- und/oder Regeleinheit zumindest ein Speicherelement aufweist, auf welchem zumindest ein Datenprofil hinterlegt ist, und wonach die Dentalbilderfassungsvorrichtung zumindest eine Sensoreinheit aufweist, welche mit der zumindest einen Steuer- und/oder Regeleinheit verbunden ist, wobei die zumindest eine Steuer- und/oder Regeleinheit dazu vorgesehen ist, abhängig von einem Signal der zumindest einen Sensoreinheit, selbsttätig ein auf dem zumindest einen Speicherelement der zumindest einen Steuer- und/oder Regeleinheit hinterlegtes Datenprofil auszuwählen, denkbar, sodass die Merkmale insbesondere auch unabhängig betrachtet zu einer Erfüllung der ausgeführten Vorteile verwendet werden können.

Zudem wird vorgeschlagen, dass die zumindest eine Steuer- und/oder Regeleinheit dazu vorgesehen ist, abhängig von zumindest einem Parameter zumindest einen Bildbearbeitungsparameter auf ein digitales Bildsignal der Kameraeinheit anzuwenden. Vorzugsweise ist die zumindest eine Steuer- und/oder Regeleinheit dazu vorgesehen, zumindest einen Bildbearbeitungsparameter auf ein digitales Bildsignal der Kameraeinheit anzuwenden, wobei ein Wert des zumindest einen Bildbearbeitungsparameters abhängig von zumindest einem Parameter variiert. Besonders bevorzugt ist die zumindest eine Steuer- und/oder Regeleinheit dazu vorgesehen, mehrere Bildbearbeitungsparameter auf ein digitales Bildsignal der Kameraeinheit anzuwenden. Unter einem "Bildbearbeitungsparameter" soll in diesem Zusammenhang insbesondere ein definierter Wert eines Bildkorrekturfaktors verstanden werden. Vorzugsweise soll darunter insbesondere ein definierter Wert eines Bildkorrekturfaktors verstanden werden, welcher mittels digitaler Bildbearbeitung auf ein digitales Bild, insbesondere ein digitales Bildsignal, der Kameraeinheit, anwendbar ist. Es sind verschiedene, einem Fachmann als sinnvoll erscheinende Bildkorrekturfaktoren denkbar, wie insbesondere eine Helligkeit, ein Kontrast, eine Sättigung und/oder ein Farbanteil. Dadurch kann vorteilhaft eine Ausgabequalität angepasst werden. Ferner können so insbesondere Störeinflüsse vermieden bzw. korrigiert werden. Vorteilhaft können Reflektionen vermieden bzw. korrigiert werden.

In einer alternativen Ausgestaltung sind diese Merkmale der Erfindung, wonach die zumindest eine Steuer- und/oder Regeleinheit dazu vorgesehen ist, abhängig von zumindest einem Parameter zumindest einen Bildbearbeitungsparameter auf ein digitales Bildsignal der Kameraeinheit anzuwenden, insbesondere in Kombination mit den Merkmalen, wonach die Dentalbilderfassungsvorrichtung eine Kameraeinheit, mit zumindest zwei Kameramodulen, zur Aufnahme stereoskopischer Bilder und zumindest eine Steuer- und/oder Regeleinheit aufweist, die Signale der Kameraeinheit verarbeitet und zumindest ein Ausgabesignal liefert, das dazu vorgesehen ist, einer Anzeigeeinheit zugeführt zu werden, auch unabhängig von den Merkmalen der Erfindung, wonach die zumindest eine Steuer- und/oder Regeleinheit zumindest ein Speicherelement aufweist, auf welchem zumindest ein Datenprofil hinterlegt ist, und wonach die Dentalbilderfassungsvorrichtung zumindest eine Sensoreinheit aufweist, welche mit der zumindest einen Steuer- und/oder Regeleinheit verbunden ist, wobei die zumindest eine Steuer- und/oder Regeleinheit dazu vorgesehen ist, abhängig von einem Signal der zumindest einen Sensoreinheit, selbsttätig ein auf dem zumindest einen Speicherelement der zumindest einen Steuer- und/oder Regeleinheit hinterlegtes Datenprofil auszuwählen, denkbar, sodass die Merkmale insbesondere auch unabhängig betrachtet zu einer Erfüllung der ausgeführten Vorteile verwendet werden können.

Zudem wird vorgeschlagen, dass die zumindest eine Steuer- und/oder Regeleinheit dazu vorgesehen ist, abhängig von zumindest einem Parameter eine Gradationskurve auf ein digitales Bildsignal der Kameraeinheit anzuwenden. Vorzugsweise ist die zumindest eine Steuer- und/oder Regeleinheit dazu vorgesehen, abhängig von zumindest einem Parameter eine Farbkurve auf ein digitales Bildsignal der Kameraeinheit anzuwenden. Vorzugsweise wird, um die Übersteuerung an den Glanzstellen zu minimieren, die Belichtungszeit stark reduziert. Hierdurch stehen die Glanzstellen insbesondere kurz vor der Übersteuerung, der Rest des Bildes ist jedoch stark abgedunkelt. Um die nun zu dunklen Stellen wieder anzuheben, wird vorzugsweise eine Gradationskurve auf ein digitales Bildsignal der Kameraeinheit angewendet. Hierbei werden insbesondere dunkle Pixel deutlich stärker angehoben als bereits helle Pixel. Über die Gradationskurve kann einem Eingangstonwert des digitalen Bildsignals ein neuer Ausgangstonwert zugeordnet werden. So können beispielsweise helle Bereiche abgedunkelt und dunkle Bereiche aufgehellt werden. Dadurch kann vorteilhaft eine Ausgabequalität angepasst werden. Eine Ausgabequalität kann vorteilhaft an Parameter angepasst werden. Insbesondere kann so eine für einen Bediener vorteilhafte Ausgabe erzeugt werden. Vorzugsweise können so insbesondere Störeinflüsse korrigiert werden. Vorteilhaft können Reflektionen vermieden werden.

In einer alternativen Ausgestaltung sind diese Merkmale der Erfindung, wonach die zumindest eine Steuer- und/oder Regeleinheit dazu vorgesehen ist, abhängig von zumindest einem Parameter eine Gradationskurve auf ein digitales Bildsignal der Kameraeinheit anzuwenden, insbesondere in Kombination mit den Merkmalen, wonach die Dentalbilderfassungsvorrichtung eine Kameraeinheit, mit zumindest zwei Kameramodulen, zur Aufnahme stereoskopischer Bilder und zumindest eine Steuer- und/oder Regeleinheit aufweist, die Signale der Kameraeinheit verarbeitet und zumindest ein Ausgabesignal liefert, das dazu vorgesehen ist, einer Anzeigeeinheit zugeführt zu werden, auch unabhängig von den Merkmalen der Erfindung, wonach die zumindest eine Steuer- und/oder Regeleinheit zumindest ein Speicherelement aufweist, auf welchem zumindest ein Datenprofil hinterlegt ist, und wonach die Dentalbilderfassungsvorrichtung zumindest eine Sensoreinheit aufweist, welche mit der zumindest einen Steuer- und/oder Regeleinheit verbunden ist, wobei die zumindest eine Steuer- und/oder Regeleinheit dazu vorgesehen ist, abhängig von einem Signal der zumindest einen Sensoreinheit, selbsttätig ein auf dem zumindest einen Speicherelement der zumindest einen Steuer- und/oder Regeleinheit hinterlegtes Datenprofil auszuwählen, denkbar, sodass die Merkmale insbesondere auch unabhängig betrachtet zu einer Erfüllung der ausgeführten Vorteile verwendet werden können.

Ferner wird vorgeschlagen, dass auf dem zumindest einen Datenprofil zumindest ein Bildbearbeitungsparameter und/oder ein Verlauf einer Gradationskurve hinterlegt ist. Vorzugsweise ist auf dem zumindest einen Datenprofil zudem ein Grenzbelichtungswert hinterlegt. Vorzugsweise sind die Bildbearbeitungsparameter und/oder der Verlauf der Gradationskurve dabei auf eine vorteilhafte Ausleuchtung bei gleichzeitiger Vermeidung von Reflektionen ausgelegt. Dadurch kann insbesondere eine schnelle Zuordnung von Einstellungen und/oder Konfigurationen erreicht werden. Insbesondere kann so eine komfortable Zuordnung von Bildbearbeitungsparametern und/oder Gradationskurven erreicht werden. Vorzugsweise kann so abhängig von einer aktuellen Situation ein Datenprofil ausgewählt werden. So kann insbesondere eine schnelle Einstellung von Einstellungen und/oder Konfigurationen erreicht werden.

Des Weiteren geht die Erfindung aus von einem Verfahren zum Betrieb einer Dentalbilderfassungsvorrichtung mit einer Kameraeinheit zur Aufnahme stereoskopischer Bilder, die zumindest zwei Kameramodule aufweist, und mit zumindest einer Steuer- und/oder Regeleinheit, die Signale der Kameraeinheit verarbeitet und zumindest ein Ausgabesignal liefert, das dazu vorgesehen ist, einer Anzeigeeinheit zugeführt zu werden, wobei die zumindest eine Steuer- und/oder Regeleinheit zumindest ein Speicherelement aufweist, auf welchem zumindest ein Datenprofil hinterlegt ist.

Erfindungsgemäß wird vorgeschlagen, dass in einem initialen Schritt zu einer Erfassung eines Grenzbelichtungswerts zumindest teilautomatisiert ein Belichtungsspektrum zumindest eines Kameramoduls der Kameraeinheit zumindest teilweise durchlaufen wird. Unter einem "Grenzbelichtungswert" soll in diesem Zusammenhang insbesondere ein Belichtungswert verstanden werden, über welchem Reflektionen in einem erzeugten digitalen Bildsignal lediglich weiß dargestellt sind, d.h. im Bereich der Reflektion insbesondere keine Tiefeninformationen mehr vorhanden sind. Vorzugsweise soll darunter insbesondere ein maximaler Belichtungswert verstanden werden, bei welchem in dem erzeugten digitalen Bildsignal zumindest großflächig Schattierungen vorhanden sind und/oder schattierungsfreie Bereiche eine definierte Größe nicht überschreiten. Der Grenzbelichtungswert ist dabei insbesondere abhängig von einer aktuellen Beleuchtung und/oder einem abgelichteten Objekt. Dadurch kann vorteilhaft ein Grenzbelichtungswert erfasst werden. Ferner kann so ein vorteilhafter Belichtungswert eingestellt werden. Es kann insbesondere erreicht werden, dass eine Tiefeninformation erhalten bleibt. Insbesondere kann so eine vorteilhafte Grundlage für eine Bildbearbeitung geschaffen werden.

Es wird ferner vorgeschlagen, dass in einem initialen Schritt zumindest teilautomatisiert abhängig von einem Signal der zumindest einen Sensoreinheit selbsttätig ein auf dem zumindest einen Speicherelement der zumindest einen Steuer- und/oder Regeleinheit hinterlegtes Datenprofil ausgewählt wird. Dadurch kann insbesondere eine selbsttätige Auswahl und Einstellung eines Datenprofils erreicht werden. So kann insbesondere eine schnelle und komfortable Einstellung von Einstellungen und/oder Konfigurationen erreicht werden. Es können falsche Einstellungen vermieden werden.

Es wird weiter vorgeschlagen, dass abhängig von einem erfassten Grenzbelichtungswert ein auf dem Speicherelement hinterlegtes Datenprofil ausgewählt wird. Vorzugsweise wird von einem erfassten Grenzbelichtungswert auf ein Material rückgeschlossen. Bevorzugt wird von einem erfassten Grenzbelichtungswert und einem dazu passenden Helligkeitswert auf ein Material rückgeschlossen. Dadurch kann vorteilhaft ein passendes Datenprofil ausgewählt werden. Über den Grenzbelichtungswert kann vorteilhaft auf ein Material rückgeschlossen werden. Ferner kann eine Bildbearbeitung so vorteilhaft an einen Grenzbelichtungswert angepasst werden. Es kann eine vorteilhafte Bildbearbeitung ermöglicht werden.

Ferner wird vorgeschlagen, dass in zumindest einem Schritt abhängig von zumindest einem Parameter zumindest teilautomatisiert ein digitales Bildsignal der Kameraeinheit vor einer Erzeugung eines Ausgabesignals bearbeitet wird. Vorzugsweise wird ein digitales Bildsignal der Kameraeinheit vor einer Erzeugung eines Ausgabesignals abhängig von zumindest einem Parameter mittels digitaler Bildbearbeitung bearbeitet. Bevorzugt wird das digitale Bildsignal der Kameraeinheit vor einer Erzeugung eines Ausgabesignals zur Vermeidung von Reflektionen abhängig von zumindest einem Parameter bearbeitet. Dadurch kann insbesondere eine vorteilhaft hohe Bildqualität erreicht werden. Insbesondere kann eine hohe Ausgabequalität erreicht werden. Ferner können Störeinflüsse vermieden bzw. korrigiert werden. Vorteilhaft können Reflektionen vermieden werden, insbesondere ohne eine Bildhelligkeit zu beeinträchtigen.

In einer alternativen Ausgestaltung sind diese Merkmale der Erfindung, wonach in zumindest einem Schritt abhängig von zumindest einem Parameter zumindest teilautomatisiert ein digitales Bildsignal der Kameraeinheit vor einer Erzeugung eines Ausgabesignals bearbeitet wird, auch unabhängig von den Merkmalen der Erfindung, wonach in einem initialen Schritt zu einer Erfassung eines Grenzbelichtungswerts zumindest teilautomatisiert ein Belichtungsspektrum zumindest eines Kameramoduls der Kameraeinheit zumindest teilweise durchlaufen wird, denkbar, sodass die Merkmale insbesondere auch unabhängig betrachtet zu einer Erfüllung der ausgeführten Vorteile verwendet werden können.

Des Weiteren wird vorgeschlagen, dass in zumindest einem Schritt abhängig von zumindest einem Parameter zumindest teilautomatisiert eine Gradationskurve auf ein digitales Bildsignal der Kameraeinheit angewendet wird. Dadurch wird vorteilhaft eine Ausgabequalität angepasst. Eine Ausgabequalität kann vorteilhaft an Parameter angepasst werden. Insbesondere kann so eine für einen Bediener vorteilhafte Ausgabe erzeugt werden. Vorzugsweise können so insbesondere Störeinflüsse korrigiert werden. Vorteilhaft können Reflektionen vermieden werden.

In einer alternativen Ausgestaltung sind diese Merkmale der Erfindung, wonach in zumindest einem Schritt abhängig von zumindest einem Parameter zumindest teilautomatisiert eine Gradationskurve auf ein digitales Bildsignal der Kameraeinheit angewendet wird, auch unabhängig von den Merkmalen der Erfindung, wonach in einem initialen Schritt zu einer Erfassung eines Grenzbelichtungswerts zumindest teilautomatisiert ein Belichtungsspektrum zumindest eines Kameramoduls der Kameraeinheit zumindest teilweise durchlaufen wird, denkbar, sodass die Merkmale insbesondere auch unabhängig betrachtet zu einer Erfüllung der ausgeführten Vorteile verwendet werden können.

Die erfindungsgemäße Dentalbilderfassungsvorrichtung sowie das erfindungsgemäße Verfahren sollen hierbei nicht auf die oben beschriebene Anwendung und Ausführungsform beschränkt sein. Insbesondere können die erfindungsgemäße Dentalbilderfassungsvorrichtung sowie das erfindungsgemäße Verfahren zu einer Erfüllung einer hierin beschriebenen Funktionsweise eine von einer hierin genannten Anzahl von einzelnen Elementen, Bauteilen und Einheiten abweichende Anzahl aufweisen.

### Zeichnungen

Weitere Vorteile ergeben sich aus der folgenden Zeichnungsbeschreibung. In den Zeichnungen sind zwei Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: ein System mit einer stereoskopischen Anzeigeeinheit und einer erfindungsgemäßen Dentalbilderfassungsvorrichtung in einer schematischen Darstellung,
- Fig. 2: das System mit der erfindungsgemäßen Dentalbilderfassungsvorrichtung in einer schematischen Schnittdarstellung durch die Schnittlinie II,
- Fig. 3: ein schematisches Ablaufdiagramm eines Verfahrens zu einem Betrieb der erfindungsgemäßen Dentalbilderfassungsvorrichtung,
- Fig. 4: eine beispielhafte Gradationskurve eines auf einem Speicherelement der erfindungsgemäßen Dentalbilderfassungsvorrichtung hinterlegten Datenprofils und
- Fig. 5: ein schematisches Ablaufdiagramm eines Verfahrens zu einem Betrieb einer alternativen erfindungsgemäßen Dentalbilderfassungsvorrichtung.

### Beschreibung der Ausführungsbeispiele

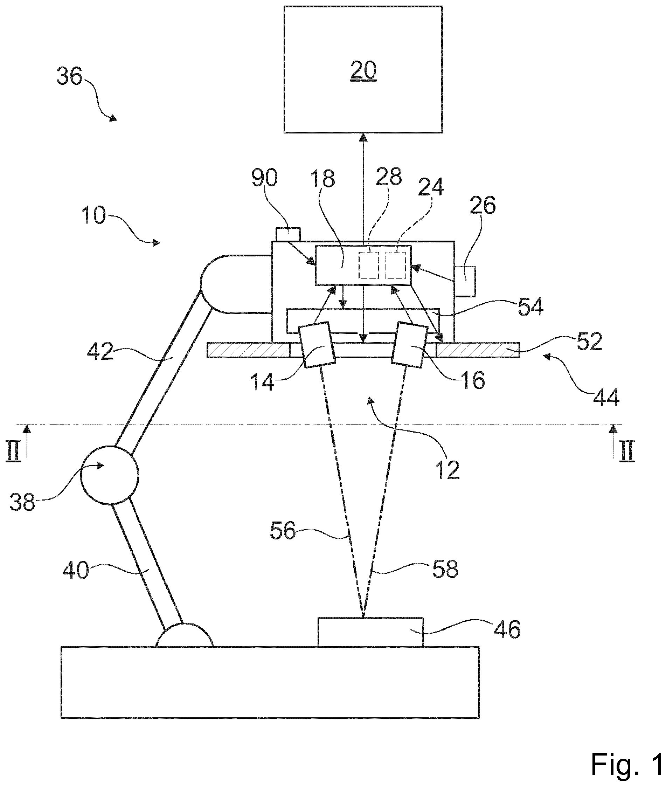

Figur 1 zeigt in schematischer Darstellung ein System 36 mit einer stereoskopischen Anzeigeeinheit 20 und einer Dentalbilderfassungsvorrichtung 10. Die Dentalbilderfassungsvorrichtung 10 ist als eine Dentaltechnikbilderfassungsvorrichtung ausgebildet. Die Dentalbilderfassungsvorrichtung 10 weist eine Kameraeinheit 12 zur Aufnahme stereoskopischer Bilder auf. Die Kameraeinheit 12 weist ein erstes Kameramodul 14 und ein zweites Kameramodul 16 auf. Die Kameramodule 14, 16 weisen jeweils einen CCD-Sensor auf. Ferner weist die Dentalbilderfassungsvorrichtung 10 eine Steuer- und Regeleinheit 18 auf, die Signale der Kameraeinheit 12 verarbeitet. Ferner liefert die Steuer- und Regeleinheit 18 ein Ausgabesignal, das dazu vorgesehen ist, der Anzeigeeinheit 20 zugeführt zu werden. Die Steuer- und Regeleinheit 18 ist bei montiertem ersten Kameramodul 14 und montiertem zweiten Kameramodul 16 dazu vorgesehen, die Signale der Kameraeinheit 12 zu verarbeiten und ein Ausgabesignal zu liefern, das dazu vorgesehen ist, der Anzeigeeinheit 20 zugeführt zu werden. Die Anzeigeeinheit 20 ist als stereoskopisches Display ausgebildet, das dazu vorgesehen ist, mit einer Shutterbrille des Systems 36 zusammenzuarbeiten, um den stereoskopischen Eindruck zu erzeugen. Alternativ sind Ausgestaltungen eines stereoskopischen Displays mit Polarisationsfiltern, die mit Polarisationsbrillen zusammenarbeiten, oder Ausgestaltungen eines stereoskopischen Displays, die ganz auf eine zusätzliche Brille verzichten können, denkbar.

Die Dentalbilderfassungsvorrichtung 10 weist ein Stativ 38 auf, das von zwei gekoppelten Schwenkarmen 40, 42 gebildet ist. Die Kameraeinheit 12 kann über das Stativ 38 in einer Höhe elektrisch verstellt werden. Ferner weist die Dentalbilderfassungsvorrichtung 10 eine Beleuchtungseinheit 44 auf, die dazu vorgesehen ist, ein zu beobachtendes Objekt 46 auszuleuchten. Die Beleuchtungseinheit 44 weist mehrere Beleuchtungselemente 48, 50 auf. Die Beleuchtungselemente 48, 50 sind in einen Beleuchtungsträger 52 der Beleuchtungseinheit 44 integriert. Die Beleuchtungselemente 48, 50 sind jeweils von LED-Modulen gebildet. Vier der Beleuchtungselemente 48 der Beleuchtungseinheit 44 sind in einem Nahbereich um die Kameraeinheit 12 angeordnet. Ferner sind vier weitere Beleuchtungselemente 50 der Beleuchtungseinheit 44 in einem Fernbereich der Kameraeinheit 12 angeordnet. Die Leuchtpegel der Beleuchtungselemente 48, 50 der Beleuchtungseinheit 44 verlaufen jeweils durch einen gemeinsamen Punkt. Grundsätzlich wäre jedoch auch eine andere, einem Fachmann als sinnvoll erscheinende Ausgestaltung der Beleuchtungseinheit 44 denkbar, wie beispielsweise aus einem Ring von LED's.

Weiterhin weist die Kameraeinheit 12 eine Aktoreinheit 54 auf, die dazu vorgesehen ist, eine Lage der Kameramodule 14, 16 zueinander in Abhängigkeit von Signalen der Steuer- und Regeleinheit 18 zu verändern. Die Steuer- und Regeleinheit 18 ist dazu vorgesehen, eine Lage der Kameramodule 14, 16 zueinander in Abhängigkeit von einem Abstand der Kameraeinheit 12 zu dem zu beobachtenden Objekt 46 anzupassen. Die Steuer- und Regeleinheit 18 ist dazu vorgesehen, eine Lage der Kameramodule 14, 16 zu korrigieren, sodass sich optische Achsen 56, 58 der Kameramodule 14, 16 in einem gemeinsamen Auftreffpunkt auf dem zu beobachtenden Objekt 46 schneiden.

Die Steuer- und Regeleinheit 18 weist ein Speicherelement 24 auf, auf welchem mehrere differierende Datenprofile hinterlegt sind. Auf dem Speicherelement 24 der Steuer- und Regeleinheit 18 sind mehrere zur Auswahl stehende Datenprofile hinterlegt. Auf dem Speicherelement 24 sind mehrere Datenprofile hinterlegt, die jeweils einem Material zugeordnet sind. Auf dem Speicherelement 24 sind mehrere Datenprofile hinterlegt, die jeweils einem Material des zu beobachtenden Objekts 46 zugeordnet sind. Es sind verschiedene, einem Fachmann als sinnvoll erscheinende Materialien denkbar, wie beispielsweise Gold und/oder Zirkonoxid. Die Datenprofile, welche einem Material des zu beobachtenden Objekts 46 zugeordnet sind, sind dabei auf eine vorteilhafte Ausleuchtung und Darstellung des Materials bei gleichzeitiger Vermeidung von Reflektionen ausgelegt. Ferner sind auf dem Speicherelement 24 mehrere Datenprofile hinterlegt, die jeweils einem Anwendungsgebiet zugeordnet sind. Des Weiteren sind auf dem Speicherelement 24 mehrere Datenprofile hinterlegt, die jeweils einem Bediener zugeordnet sind, der persönliche Einstellungen speichern kann. Es wird daher zwischen Materialdatenprofilen, Anwendungsdatenprofilen und Nutzerdatenprofilen unterschieden. Grundsätzlich wären auch andere, einem Fachmann als sinnvoll erscheinende Datenprofile oder lediglich ein Teil der Datenprofile denkbar. Die Kombination von Datenprofilen ist hierbei lediglich beispielhaft.

Die Steuer- und Regeleinheit 18 ist dazu vorgesehen, abhängig von einem ausgewählten, auf dem Speicherelement 24 hinterlegten Datenprofil die Signale der Kameraeinheit 12 und ein Ausgabesignal anzupassen. Die Steuer- und Regeleinheit 18 ist dazu vorgesehen, abhängig von einem Parameter, ein digitales Bildsignal der Kameraeinheit 12 vor einer Erzeugung eines Ausgabesignals zu bearbeiten. Die Steuer- und Regeleinheit 18 ist dazu vorgesehen, abhängig von einem ausgewählten, auf dem Speicherelement 24 hinterlegten Datenprofil ein digitales Bildsignal der Kameraeinheit 12 vor einer Erzeugung eines Ausgabesignals zu bearbeiten. Der Parameter ist von einem ausgewählten, auf dem Speicherelement 24 hinterlegten Datenprofil gebildet. Die Steuer- und Regeleinheit 18 ist dazu vorgesehen, zur Vermeidung von Reflektionen, abhängig von einem ausgewählten, auf dem Speicherelement 24 hinterlegten Datenprofil ein digitales Bildsignal der Kameraeinheit 12 vor einer Erzeugung eines Ausgabesignals mittels digitaler Bildbearbeitung zu bearbeiten. Eine Bearbeitung erfolgt dabei in Echtzeit. Die Steuer- und Regeleinheit 18 ist zu einer Bildbearbeitung dazu vorgesehen, abhängig von einem ausgewählten, auf dem Speicherelement 24 hinterlegten Datenprofil Bildbearbeitungsparameter auf ein digitales Bildsignal der Kameraeinheit 12 anzuwenden. Die Steuer- und Regeleinheit 18 ist dazu vorgesehen, abhängig von einem ausgewählten, auf dem Speicherelement 24 hinterlegten Datenprofil eine Gradationskurve 22a, 22a' auf ein digitales Bildsignal der Kameraeinheit 12 anzuwenden. Der Parameter ist von einem ausgewählten Profil gebildet.

Auf einem Teil der Datenprofile ist ein Verlauf einer Gradationskurve 22a, 22a' hinterlegt. Die Gradationskurven 22a, 22a' sind zu einer Verringerung eines Rechenaufwands, als Lookup-Tabellen hinterlegt. Auf den Datenprofilen, die jeweils einem Material zugeordnet sind, und auf den Datenprofilen, die jeweils einem Anwendungsgebiet zugeordnet sind, ist jeweils ein Verlauf einer Gradationskurve 22a, 22a' hinterlegt. Die Gradationskurven 22a, 22a' sind jeweils an das Material und/oder das Anwendungsgebiet angepasst. Zudem sind auf den Datenprofilen je nach Zuordnung auch noch weitere Informationen hinterlegt. Dabei sind auf den Materialdatenprofilen der Datenprofile jeweils ein Verlauf einer Gradationskurve 22a, 22a', ein Helligkeitsfaktor und eine Beleuchtungseinstellung für die Beleuchtungseinheit 44 hinterlegt. Auf den Anwendungsdatenprofilen der Datenprofile sind jeweils ein Verlauf einer Gradationskurve 22a, 22a', ein Helligkeitsfaktor und eine Beleuchtungseinstellung für die Beleuchtungseinheit 44 hinterlegt. Ferner sind auf den Nutzerdatenprofilen der Datenprofile jeweils eine Beleuchtungseinstellung für die Beleuchtungseinheit 44 sowie eine Lageeinstellung der Kameraeinheit 12 hinterlegt. Grundsätzlich sind jedoch auch zusätzliche oder alternative auf den Datenprofilen hinterlegte Informationen und/oder Einstellungen denkbar.

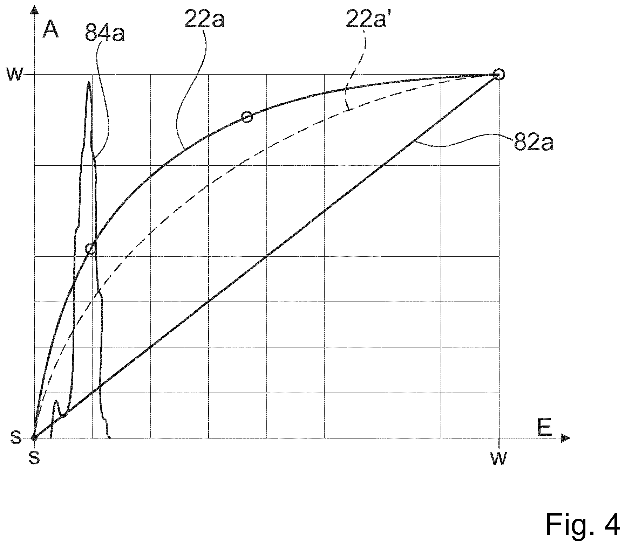

Zu einer Vereinfachung wird lediglich zwischen verschiedenen Stufen von Gradationskurven 22a, 22a' unterschieden. Die Stufe 0 bildet dabei eine Ausgangsgerade 82a. Die Ausgangsgerade 82a definiert ein unverändertes Bild, bei dem Ausgangstonwert und Eingangstonwert identisch sind. Mit zunehmender Stufe steigt die Krümmung der Gradationskurven 22a, 22a'. Beispielhaft ist in einem Datenprofil des Materials Gold eine Stufe 3 Gradationskurve 22a hinterlegt. Die Stufe 3 Gradationskurve 22a entspricht der Gradationskurve 22a in der Figur 4. Ferner ist in einem Datenprofil des Materials Zirkonoxid beispielhaft eine Stufe 2 Gradationskurve 22a' hinterlegt. Diese weist gegenüber der Stufe 3 Gradationskurve 22a eine geringe Krümmung auf. Die Stufe 2 Gradationskurve 22a' entspricht der gestrichelten Gradationskurve 22a' in der Figur 4. Es wird insbesondere lediglich zwischen den Stufen 0 bis 5 von Gradationskurven unterschieden. Des Weiteren ist in einem Datenprofil des Materials Zirkonoxid beispielhaft eine Beleuchtungseinstellung für die Beleuchtungseinheit 44 hinterlegt. Bei der Beleuchtungseinstellung des Datenprofils wird ein hinteres der Beleuchtungselemente 48 der Beleuchtungseinheit 44, welche in einem Nahbereich um die Kameraeinheit 12 angeordnet sind, deaktiviert. Die drei restlichen Beleuchtungselemente 48 in dem Nahbereich werden aktiviert. Ferner werden die vier weiteren Beleuchtungselemente 50 der Beleuchtungseinheit 44, die in einem Fernbereich der Kameraeinheit 12 angeordnet sind, ebenfalls aktiviert. Durch diese Beleuchtung können vorteilhaft Reflektionen vermieden werden. Grundsätzlich ist jedoch auch eine andere, einem Fachmann als sinnvoll erscheinende Beleuchtungseinstellung denkbar.

Die auf den Datenprofilen hinterlegten Helligkeitsfaktoren können einen Wert zwischen 0 und 255 aufweisen. Ein Datenprofil des Materials Gold weist beispielsweise einen Helligkeitsfaktor mit dem Wert 112 auf. Ein Datenprofil des Materials Zirkonoxid weist dagegen beispielsweise einen Helligkeitsfaktor mit dem Wert 144 auf. Der Helligkeitsfaktor definiert dabei einen Durchschnittlichen Helligkeitswert eines digitalen Bildsignals. Zu einer Erfassung eines aktuellen durchschnittlichen Helligkeitswert wird während eines Betriebs in einem definierten Messbereich der CCD-Sensoren der Kameramodule 14, 16 ein durchschnittlicher Wert der Helligkeitswerte der einzelnen Pixel in diesem Messbereich ermittelt. Zu einer Anpassung des durchschnittlichen Helligkeitswerts an den Helligkeitsfaktor wird ein aktueller durchschnittlicher Helligkeitswert durch Anpassung einer Belichtungszeit der Kameramodule 14, 16 dem Helligkeitsfaktor angeglichen, bis der aktuelle durchschnittliche Helligkeitswert und der Helligkeitsfaktor identisch sind. Liegt ein aktueller Helligkeitswert unter einem eingestellten Helligkeitsfaktor, wird eine Belichtungszeit erhöht. Liegt ein aktueller Helligkeitswert über einem eingestellten Helligkeitsfaktor, wird eine Belichtungszeit reduziert. Dies wird wiederholt, bis der aktuelle Helligkeitswert dem Helligkeitsfaktor entspricht. Die Helligkeitswerte der einzelnen Pixel der CCD-Sensoren liegen ebenfalls zwischen 0 und 255.

Ferner weist die Dentalbilderfassungsvorrichtung 10 eine Eingabeeinheit 26 zu einer Eingabe des Parameters auf. Die Eingabeeinheit 26 ist zu einer Auswahl eines auf dem Speicherelement 24 der Steuer- und Regeleinheit 18 hinterlegten Datenprofils vorgesehen. Über die Eingabeeinheit 26 kann ein Bediener ein gewünschtes Datenprofil auswählen. Es kann dabei sowohl ein Datenprofil für ein verwendetes Material, für ein aktuelles Anwendungsgebiet als auch ein von dem Bediener selbst erstelltes Nutzerdatenprofil ausgewählt werden. Über die Eingabeeinheit 26 kann auch ein Datenprofil, insbesondere ein Nutzerdatenprofil, bearbeitet oder angelegt werden. Die Eingabeeinheit 26 ist mit der Steuer- und Regeleinheit 18 verbunden. Die Eingabeeinheit 26 ist als ein Tastenfeld ausgebildet. Über die Eingabeeinheit 26 kann zwischen Materialdatenprofilen, Anwendungsdatenprofilen und Nutzerdatenprofilen ausgewählt werden. Grundsätzlich wäre jedoch auch eine andere, einem Fachmann als sinnvoll erscheinende Ausbildung der Eingabeeinheit 26 denkbar, wie beispielsweise als ein Touchpad oder ein Touchscreen. Ein Menü zur Auswahl eines Datenprofils wird über die Steuer- und Regeleinheit 18 auf der Anzeigeeinheit 20 ausgegeben. Grundsätzlich wäre jedoch auch denkbar, dass ein entsprechendes Menü auf einem separaten Display ausgegeben wird.

Des Weiteren weist die Dentalbilderfassungsvorrichtung 10 eine Sensoreinheit 28 auf, welche mit der zumindest einen Steuer- und Regeleinheit 18 verbunden ist. Die Sensoreinheit 28 ist in die Steuer- und Regeleinheit 18 integriert. Die Steuer- und Regeleinheit 18 ist dazu vorgesehen, abhängig von einem Signal der Sensoreinheit 28, selbsttätig ein auf dem Speicherelement 24 der Steuer- und Regeleinheit 18 hinterlegtes Datenprofil auszuwählen. Wird über die Eingabeeinheit 26 von einem Bediener kein Datenprofil ausgewählt, wird von der Sensoreinheit 28 ein verwendetes Material sensiert und von der Steuer- und Regeleinheit 18 ein dem Material zugeordnetes Datenprofil ausgewählt. So kann verhindert werden, dass ein Betrieb ohne ein ausgewähltes Datenprofil durchgeführt wird. Ferner kann vermieden werden, dass ein Bediener zwangsläufig ein Datenprofil auswählen muss. Es kann ein hoher Komfort erreicht werden. Wird ein Datenprofil selbsttätig von der Steuer- und Regeleinheit 18 ausgewählt, wird grundsätzlich eines der Datenprofile ausgewählt, die jeweils einem Material zugeordnet sind. Zu einer Erfassung eines Materials, ist die Sensoreinheit 28 dazu vorgesehen, ein Grenzbelichtungswert des Materials zu erfassen. Hierbei muss grundsätzlich nicht das exakte Material erfasst werden. Es genügt hierbei insbesondere ein Spiegelungsverhalten des Materials. Insbesondere auch, da bei Materialverbunden kein definiertes Material erkannt werden kann. Dabei wäre insbesondere auch denkbar, dass bei einer Auswahl eines Datenprofils abhängig von einem Signal der Sensoreinheit 28, ein neues, individuelles Datenprofil von der Steuer- und Regeleinheit 18 berechnet und angelegt wird. Dadurch kann ein optimales Ausgabeergebnis erzielt werden. Ferner weist die Dentalbilderfassungsvorrichtung 10 eine Schnittstelle 90 auf. Die Schnittstelle 90 ist als Datenschnittstelle ausgebildet. Die Schnittstelle 90 ist von einer USB-Schnittstelle gebildet. Grundsätzlich wäre jedoch auch eine andere, einem Fachmann als sinnvoll erscheinende Ausbildung denkbar. Über die Schnittstelle 90 kann ein externes Datenprofil auf das Speicherelement 24 der Steuer- und Regeleinheit 18 übertragen werden. Über die Schnittstelle 90 können zusätzliche, beispielsweise selbst erstellte Datenprofile oder Datenprofile von weiteren erfindungsgemäßen Dentalbilderfassungsvorrichtungen auf das Speicherelement 24 der Steuer- und Regeleinheit 18 übertragen werden, wo sie von der Steuer- und Regeleinheit 18 ausgewählt werden können. Über die Schnittstelle 90 können auch Datenprofile vom Speicherelement 24 der Steuer- und Regeleinheit 18 herunterkopiert werden.

Des Weiteren können Datenprofile auch manuell angelegt und erstellt werden. Dies kann sowohl über die Eingabeeinheit 26 erfolgen als auch extern. Extern erstellte Datenprofile können über die Schnittstelle 90 auf das Speicherelement 24 übertragen werden. Bei einer externen Erstellung eines Datenprofils kann ein unbearbeitetes Bildsignal verwendet werden, um eine optimale Gradationskurve 22a, 22a' auszuwählen bzw. zu erstellen. Hierzu kann eine Gradationskurve 22a, 22a' auf unbearbeitete Bildsignale angewendet werden, welche in Echtzeit bearbeitet werden. Dadurch kann eine optimale Gradationskurve 22a, 22a' erstellt werden.

Zudem bietet die Dentalbilderfassungsvorrichtung 10 die Möglichkeit, Momentaufnahmen zu erstellen. Die Momentaufnahmen können über die Schnittstelle 90 oder direkt über eine Internetverbindung auf externe Geräte übertragen werden. So können Momentaufnahmen beispielsweise an einen Zahnarzt oder einen Zahntechniker übertragen werden. Die Steuer- und Regeleinheit 18 ist dazu vorgesehen, eine Momentaufnahme mit einem aktuell ausgewählten Datenprofil zu verknüpfen. Die Steuer- und Regeleinheit 18 hinterlegt auf einer erzeugten Datei der Momentaufnahme zusätzlich ein aktuell ausgewähltes Datenprofil. Durch die Verknüpfung mit dem aktuell ausgewählten Datenprofil kann erreicht werden, dass ein Empfänger nachvollziehen kann, unter welchen Bedingungen die Momentaufnahme gemacht wurde. So kann beispielsweise auch ein Originalzustand der Momentaufnahme rekonstruiert werden.

Im Folgenden ist ein Verfahren zum Betrieb der Dentalbilderfassungsvorrichtung 10 beschrieben.

Nach einem Start 60a der Dentalbilderfassungsvorrichtung 10 kann in einem initialen Schritt 62a über die Eingabeeinheit 26 ein Datenprofil ausgewählt werden. Es kann dabei zwischen verschiedenen Materialdatenprofilen, Anwendungsdatenprofilen und Nutzerdatenprofilen ausgewählt werden. Es kann dabei nur ein Datenprofil ausgewählt werden. Grundsätzlich wäre jedoch auch denkbar, dass mehrere Datenprofile aus verschiedenen Gruppen ausgewählt werden können. Anschließend wird von der Steuer- und Regeleinheit 18 in einem weiteren Schritt 64a überprüft, ob ein Datenprofil von einem Bediener ausgewählt wurde. Grundsätzlich wäre jedoch auch denkbar, dass ein Bediener durch eine einfache Eingabe bestätigen muss, wenn er kein Datenprofil auswählen möchte.

Wurde kein Datenprofil ausgewählt, wird in einem initialen Schritt 34a ein Grenzbelichtungswert sensiert. In dem initialen Schritt 34a wird, zu einer Erfassung des Grenzbelichtungswerts, automatisiert ein Belichtungsspektrum der Kameramodule 14, 16 der Kameraeinheit 12 durchlaufen. Dabei wird mit einer geringsten Belichtungszeit gestartet, die langsam angehoben wird. Grundsätzlich wäre jedoch auch denkbar, dass eine Belichtung über eine Öffnungsgröße einer Blende der Kameramodule 14, 16 der Kameraeinheit 12 gesteuert wird. Während dem Durchlaufen des Belichtungsspektrums, wird an einem digitalen Bildsignal der Kameraeinheit 12 über die Sensoreinheit 28 überprüft, in welchem Bereich des digitalen Bildsignals Reflektionen auftreten. Tritt in dem Bereich der Reflektion eine weiße Fläche auf, wurde der Grenzbelichtungswert aktuell überschritten. Daher bildet ein vorheriger Belichtungswert den Grenzbelichtungswert. Anschließend wird in einem weiteren Schritt 66a zu dem Grenzbelichtungswert ein durchschnittlicher Helligkeitswert in einem definierten Messbereich der CCD-Sensoren der Kameramodule 14, 16 erfasst. Abhängig von dem erfassten durchschnittlichen Helligkeitswert wird ein auf dem Speicherelement 24 hinterlegtes Datenprofil ausgewählt. In dem initialen Schritt 66a wird automatisiert abhängig von einem Signal der Sensoreinheit 28 selbsttätig ein auf dem Speicherelement 24 der zumindest einen Steuer- und Regeleinheit 18 hinterlegtes Datenprofil ausgewählt. Dazu wird der erfasste Helligkeitswert mit den auf dem Speicherelement 24 hinterlegten Materialdatenprofilen der Datenprofile verglichen. Das Materialdatenprofil, dessen Helligkeitsfaktor dem erfassten Helligkeitswert am nächsten kommt, wird ausgewählt. Grundsätzlich wäre jedoch auch denkbar, dass der erfasste Helligkeitswert genutzt wird und nicht der Helligkeitswert an den Helligkeitsfaktor des ausgewählten Materialdatenprofils angeglichen wird. Anschließend folgt ein Schritt 70a.

Wird in dem Schritt 64a festgestellt, dass in dem Schritt 62a ein Datenprofil ausgewählt wurde, wird in einem weiteren Schritt 72a überprüft, ob ein Nutzerdatenprofil ausgewählt wurde. Wurde kein Nutzerdatenprofil ausgewählt, folgt der Schritt 70a.

Wurde ein Nutzerdatenprofil ausgewählt, wird in einem weiteren Schritt 74a eine auf dem ausgewählten Datenprofil hinterlegte Lageeinstellung der Kameraeinheit 12 ausgelesen und über das Stativ 38 eingestellt. Die Kameraeinheit 12 kann so auf eine von dem Bediener gewünschte Höhe eingestellt werden. So kann insbesondere erreicht werden, dass die Kameraeinheit 12 weder bei einem Bearbeiten eines beobachteten Objekts 46 noch bei einem Blick auf die Anzeigeeinheit 20 stört. Es können so individuelle Wünsche eingestellt werden. Anschließend wird in einem weiteren Schritt 76a, der identisch zu dem Schritt 34a ist, ein Grenzbelichtungswert sensiert. Danach wird in einem weiteren Schritt 78a abhängig von dem erfassten Grenzbelichtungswert ein auf dem Speicherelement 24 hinterlegtes Datenprofil ausgewählt. Dazu wird zu dem erfassten Grenzbelichtungswert ein durchschnittlicher Helligkeitswert in einem definierten Messbereich der CCD-Sensoren der Kameramodule 14, 16 erfasst. Abhängig von dem erfassten durchschnittlichen Helligkeitswert wird ein auf dem Speicherelement 24 hinterlegtes Datenprofil ausgewählt. Dabei wird automatisiert abhängig von einem Signal der Sensoreinheit 28 selbsttätig ein auf dem Speicherelement 24 der zumindest einen Steuer- und Regeleinheit 18 hinterlegtes Datenprofil ausgewählt. Dazu wird der erfasste Helligkeitswert mit den auf dem Speicherelement 24 hinterlegten Materialdatenprofilen der Datenprofile verglichen. Das Materialdatenprofil, dessen Helligkeitsfaktor dem erfassten Helligkeitswert am nächsten kommt, wird ausgewählt. Grundsätzlich wäre jedoch auch denkbar, dass der erfasste Helligkeitswert genutzt wird und nicht der Helligkeitswert an den Helligkeitsfaktor des ausgewählten Materialdatenprofils angeglichen wird. Anschließend folgt der Schritt 70a.

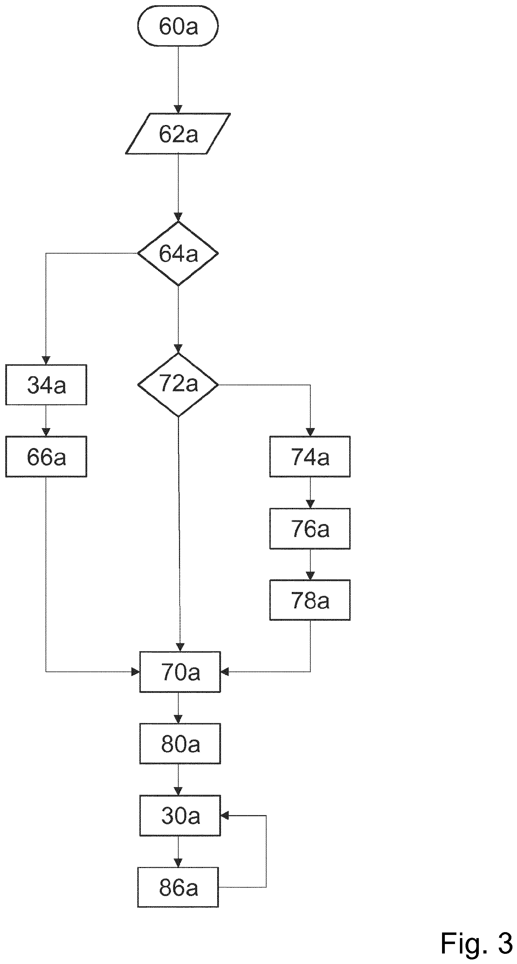

Mit dem Schritt 70a beginnt ein regulärer Betrieb. In dem Schritt 70a wird bei der Kameraeinheit 12 der Helligkeitsfaktor des ausgewählten Datenprofils eingestellt. In dem regulären Betrieb werden die Kameramodule 14, 16 der Kameraeinheit 12 auf den Helligkeitsfaktor eingestellt. Dazu wird der durchschnittliche Helligkeitswert auf den Helligkeitsfaktor eingestellt. Dies erfolgt durch eine Anpassung einer Belichtungszeit der Kameramodule 14, 16 bis der aktuelle durchschnittliche Helligkeitswert und der Helligkeitsfaktor identisch sind. Liegt ein aktueller Helligkeitswert unter einem eingestellten Helligkeitsfaktor wird eine Belichtungszeit erhöht. Liegt ein aktueller Helligkeitswert über einem eingestellten Helligkeitsfaktor wird eine Belichtungszeit reduziert. Dies wird wiederholt, bis der aktuelle Helligkeitswert dem Helligkeitsfaktor entspricht. Grundsätzlich wäre jedoch auch denkbar, dass der aktuelle Helligkeitswert gegenüber dem Helligkeitsfaktor leicht angehoben wird. Hierdurch könnte eine mögliche, zu starke Körnung bei einer späteren Aufhellung vermieden werden. Die Belichtungszeit wird ständig über den Helligkeitsfaktor angepasst. Grundsätzlich wäre jedoch auch denkbar, dass initial eine Belichtungszeit abhängig von einem eingestellten Helligkeitsfaktor erfasst und eingestellt wird. Anschließend wird in einem Schritt 80a die Beleuchtungseinheit 44 auf die Beleuchtungseinstellung des ausgewählten Datenprofils eingestellt. Dabei werden über die Steuer- und Regeleinheit 18 definiert einzelne Beleuchtungselemente 48, 50 der Beleuchtungseinheit 44 aktiviert und einzelne Beleuchtungselemente 48, 50 der Beleuchtungseinheit 44 deaktiviert. Die aktivierten Beleuchtungselemente 48, 50 werden von der Steuer- und Regeleinheit 18 auf einen auf dem ausgewählten Datenprofil hinterlegten Wert gedimmt. Über die Beleuchtungseinstellung kann gezielt eine Ausleuchtung gesteuert werden. Darauffolgend wird in einem weiteren Schritt 30a, welcher in der Figur 3 als Unterprogramm dargestellt ist, ein regulärer Filmbetrieb durchgeführt. Dabei werden von der Kameraeinheit 12 stereoskopische Bilder aufgenommen, die in Form digitaler Bilddateien an die Steuer- und Regeleinheit 18 übertragen werden. Die Bilder werden von der Kameraeinheit 12 mit dem Helligkeitsfaktor des ausgewählten Datenprofils aufgenommen.

Anschließend wird in dem Schritt 30a, abhängig von einem Parameter, automatisiert das digitale Bildsignal der Kameraeinheit 12 vor einer Erzeugung eines Ausgabesignals bearbeitet. Das digitale Bildsignal der Kameraeinheit 12 wird, abhängig von einem ausgewählten Datenprofil vor einer Erzeugung eines Ausgabesignals automatisiert von der Steuer- und Regeleinheit 18 bearbeitet. In dem Schritt 30a wird, abhängig von einem Parameter, automatisiert eine Gradationskurve 22a, 22a' auf das digitale Bildsignal der Kameraeinheit 12 angewendet. Die Steuer- und Regeleinheit 18 wendet die auf dem ausgewählten Datenprofil hinterlegte Gradationskurve 22a, 22a' auf das digitale Bildsignal der Kameraeinheit 12 an. Dabei wird ein digitales Bildsignal der Kameraeinheit 12 vor einer Erzeugung eines Ausgabesignals aufgehellt, wobei dunkle Pixel um einen deutlich höheren Faktor angehoben werden als helle Pixel. In der Figur 4 sind beispielhaft zwei Gradationskurven 22a, 22a' dargestellt. Dabei ist auf einer x-Achse ein ursprünglicher Eingangstonwert E des digitalen Bildsignals aufgetragen. Auf einer y-Achse ist ein neuer Ausgangstonwert A aufgetragen, welcher dem bearbeiteten Bildsignal zugeordnet wird. Die Tonwerte E, A erstrecken sich dabei jeweils von schwarz s nach weiß w. In der Figur 4 ist zu den Gradationskurven 22a, 22a' eine Ausgangsgerade 82a dargestellt, welche ein unverändertes Bild definiert. Ferner ist ein Histogramm 84a dargestellt. Aus dem Histogramm 84a geht hervor, dass ein Peak-Wert eines Eingangstonwerts am meisten angehoben wird, das heißt, dass die Mehrheit aller Pixel um den Faktor 4 - 5 verstärkt wird. Dadurch wird das stark abgedunkelte Bild aufgehellt. Aus dem bearbeiteten Bildsignal wird in einem weiteren Schritt 86a von der Steuer- und Regeleinheit 18a ein Ausgangssignal mit einer stereoskopischen Bildinformation erzeugt und der Anzeigeeinheit 20a zugeführt. Die Schritte 30a und 86a werden während eines regulären Betriebs ständig wiederholt, bis die Dentalbilderfassungsvorrichtung 10 deaktiviert wird.

Es kann dadurch eine besonders vorteilhafte Anzeige erreicht werden. Dazu wird, um die Übersteuerung an den Glanzstellen gering zu halten, die Belichtungszeit stark reduziert. Hierdurch stehen die Glanzstellen kurz vor der Übersteuerung und der Rest des Bildes ist stark abgedunkelt. Um die nun zu dunklen Stellen wieder anzuheben, wird die Operation "Kurve" auf dieses Bild angewendet, das heißt, die Gradationskurve 22a, 22a' wird auf das digitale Bildsignal angewendet. Hierbei werden dunkle Pixel deutlich stärker angehoben als bereits helle Pixel. Es entsteht ein gleichmäßig ausgeleuchtetes Bild, bei welchem auch Tiefen vorteilhaft sichtbar sind.

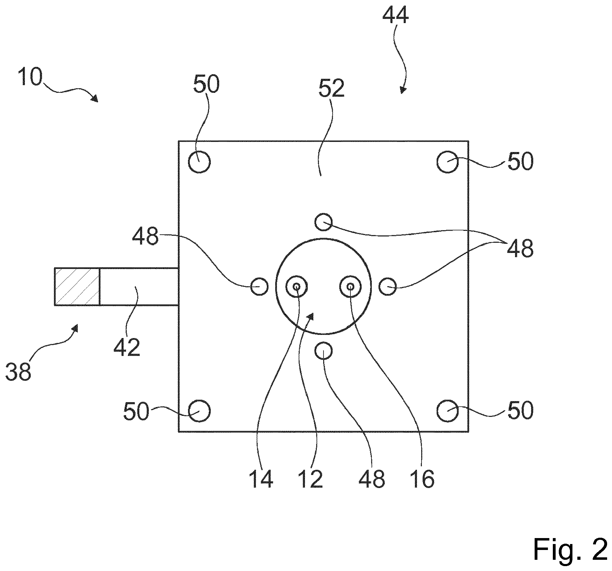

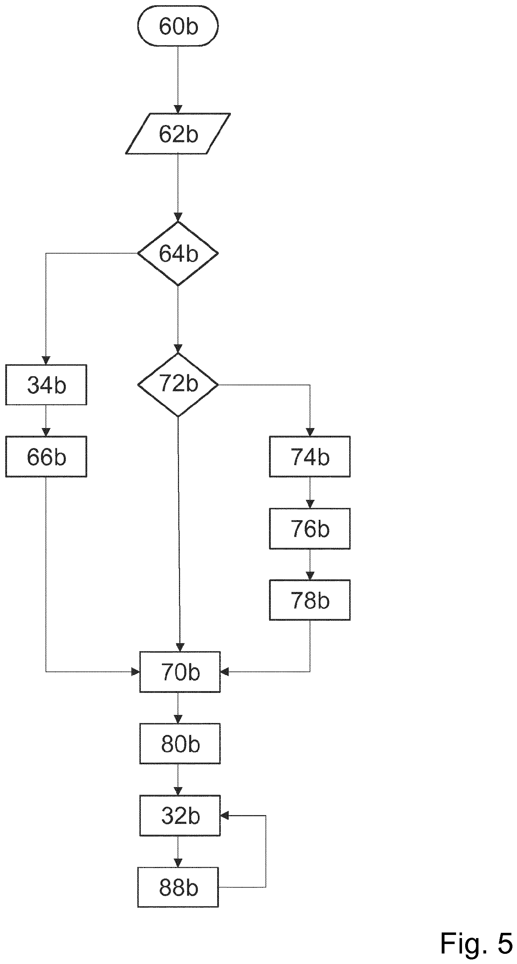

In Figur 5 ist ein weiteres Ausführungsbeispiel der Erfindung gezeigt. Die nachfolgenden Beschreibungen beschränken sich im Wesentlichen auf die Unterschiede zwischen den Ausführungsbeispielen, wobei bezüglich gleichbleibender Bauteile, Merkmale und Funktionen auf die Beschreibung des ersten Ausführungsbeispiels der Figuren 1 bis 4 verwiesen werden kann. Zur Unterscheidung der Ausführungsbeispiele ist der Buchstabe a in den Bezugszeichen des Ablaufdiagramms des Ausführungsbeispiels der Figuren 1 bis 4 durch den Buchstaben b in den Bezugszeichen des Ablaufdiagramms des zweiten Ausführungsbeispiels der Figur 5 ersetzt. Bezüglich gleich bezeichneter Bauteile, insbesondere in Bezug auf Bauteile mit gleichen Bezugszeichen, kann grundsätzlich auch auf die Zeichnungen und/oder die Beschreibung des ersten Ausführungsbeispiels der Figur 1 bis 4 verwiesen werden.

Figur 5 zeigt ein schematisches Ablaufdiagramm eines Verfahrens zu einem Betrieb einer alternativen erfindungsgemäßen Dentalbilderfassungsvorrichtung 10. Die Dentalbilderfassungsvorrichtung 10 weist eine Steuer- und Regeleinheit 18 auf. Die Steuer- und Regeleinheit 18 weist ein Speicherelement 24 auf, auf welchem mehrere differierende Datenprofile hinterlegt sind. Auf dem Speicherelement 24 der Steuer- und Regeleinheit 18 sind mehrere zur Auswahl stehende Datenprofile hinterlegt.

Auf den Datenprofilen sind je nach Zuordnung Informationen hinterlegt. Dabei sind auf Materialdatenprofilen der Datenprofile jeweils ein Helligkeitsfaktor und eine Beleuchtungseinstellung für die Beleuchtungseinheit 44 hinterlegt. Auf Anwendungsdatenprofilen der Datenprofile sind jeweils ein Helligkeitsfaktor und eine Beleuchtungseinstellung für die Beleuchtungseinheit 44 hinterlegt. Ferner sind auf den Nutzerdatenprofilen der Datenprofile jeweils eine Beleuchtungseinstellung für die Beleuchtungseinheit 44 sowie eine Lageeinstellung einer Kameraeinheit 12 hinterlegt. Grundsätzlich sind jedoch auch zusätzliche oder alternative auf den Datenprofilen hinterlegte Informationen und/oder Einstellungen denkbar.

Im Folgenden ist ein Verfahren zum Betrieb der Dentalbilderfassungsvorrichtung 10 beschrieben.

Nach einem Start 60b der Dentalbilderfassungsvorrichtung 10 kann in einem initialen Schritt 62b über die Eingabeeinheit 26 ein Datenprofil ausgewählt werden. Es kann dabei zwischen verschiedenen Materialdatenprofilen, Anwendungsdatenprofilen und Nutzerdatenprofilen ausgewählt werden. Es kann dabei nur ein Datenprofil ausgewählt werden. Grundsätzlich wäre jedoch auch denkbar, dass mehrere Datenprofile aus verschiedenen Gruppen ausgewählt werden können. Anschließend wird von der Steuer- und Regeleinheit 18 in einem weiteren Schritt 64b überprüft, ob ein Datenprofil von einem Bediener ausgewählt wurde. Grundsätzlich wäre jedoch auch denkbar, dass ein Bediener durch eine einfache Eingabe bestätigen muss, wenn er kein Datenprofil auswählen möchte.

Wurde kein Datenprofil ausgewählt, wird in einem initialen Schritt 34b ein Grenzbelichtungswert sensiert. In dem initialen Schritt 34b wird zu einer Erfassung des Grenzbelichtungswerts automatisiert ein Belichtungsspektrum der Kameramodule 14, 16 der Kameraeinheit 12 durchlaufen. Dabei wird mit einer geringsten Belichtungszeit gestartet, die langsam angehoben wird. Grundsätzlich wäre jedoch auch denkbar, dass eine Belichtung über eine Öffnungsgröße einer Blende der Kameramodule 14, 16 der Kameraeinheit 12 gesteuert wird. Während dem Durchlaufen des Belichtungsspektrums, wird an einem digitalen Bildsignal der Kameraeinheit 12 über die Sensoreinheit 28 überprüft, in welchem Bereich des digitalen Bildsignals Reflektionen auftreten. Tritt in dem Bereich der Reflektion eine weiße Fläche auf, wurde der Grenzbelichtungswert aktuell überschritten. Daher bildet ein vorheriger Belichtungswert den Grenzbelichtungswert. Anschließend wird in einem weiteren Schritt 66b zu dem Grenzbelichtungswert ein durchschnittlicher Helligkeitswert in einem definierten Messbereich der CCD-Sensoren der Kameramodule 14, 16 erfasst. Abhängig von dem erfassten durchschnittlichen Helligkeitswert, wird ein auf dem Speicherelement 24 hinterlegtes Datenprofil ausgewählt. In dem initialen Schritt 66b wird automatisiert abhängig von einem Signal der Sensoreinheit 28 selbsttätig ein auf dem Speicherelement 24 der zumindest einen Steuer- und Regeleinheit 18 hinterlegtes Datenprofil ausgewählt. Dazu wird der erfasste Helligkeitswert mit den auf dem Speicherelement 24 hinterlegten Materialdatenprofilen der Datenprofile verglichen. Das Materialdatenprofil, dessen Helligkeitsfaktor dem erfassten Helligkeitswert am nächsten kommt, wird ausgewählt. Grundsätzlich wäre jedoch auch denkbar, dass der erfasste Helligkeitswert genutzt wird und nicht der Helligkeitswert an den Helligkeitsfaktor des ausgewählten Materialdatenprofils angeglichen wird. Anschließend folgt ein Schritt 70b.

Wird in dem Schritt 64b festgestellt, dass in dem Schritt 62b ein Datenprofil ausgewählt wurde, wird in einem weiteren Schritt 72b überprüft, ob ein Nutzerdatenprofil ausgewählt wurde. Wurde kein Nutzerdatenprofil ausgewählt, folgt der Schritt 70b.

Wurde ein Nutzerdatenprofil ausgewählt, wird in einem weiteren Schritt 74b eine auf dem ausgewählten Datenprofil hinterlegte Lageeinstellung der Kameraeinheit 12 ausgelesen und über das Stativ 38 eingestellt. Die Kameraeinheit 12 kann so auf eine von dem Bediener gewünschte Höhe eingestellt werden. So kann insbesondere erreicht werden, dass die Kameraeinheit 12 weder bei einem Bearbeiten eines beobachteten Objekts 46 noch bei einem Blick auf die Anzeigeeinheit 20 stört. Es können so individuelle Wünsche eingestellt werden. Anschließend wird in einem weiteren Schritt 76a, der identisch zu dem Schritt 34b ist, ein Grenzbelichtungswert sensiert. Dazu wird zu dem erfassten Grenzbelichtungswert ein durchschnittlicher Helligkeitswert in einem definierten Messbereich der CCD-Sensoren der Kameramodule 14, 16 erfasst. Abhängig von dem erfassten durchschnittlichen Helligkeitswert wird ein auf dem Speicherelement 24 hinterlegtes Datenprofil ausgewählt. Dabei wird automatisiert abhängig von einem Signal der Sensoreinheit 28 selbsttätig ein auf dem Speicherelement 24 der zumindest einen Steuer- und Regeleinheit 18 hinterlegtes Datenprofil ausgewählt. Dazu wird der erfasste Helligkeitswert mit den auf dem Speicherelement 24 hinterlegten Materialdatenprofilen der Datenprofile verglichen. Das Materialdatenprofil, dessen Helligkeitsfaktor dem erfassten Helligkeitswert am nächsten kommt, wird ausgewählt. Grundsätzlich wäre jedoch auch denkbar, dass der erfasste Helligkeitswert genutzt wird und nicht der Helligkeitswert an den Helligkeitsfaktor des ausgewählten Materialdatenprofils angeglichen wird. Anschließend folgt der Schritt 70b.

Mit dem Schritt 70b beginnt ein regulärer Betrieb. In dem Schritt 70b wird bei der Kameraeinheit 12 der Helligkeitsfaktor des ausgewählten Datenprofils eingestellt. Dazu wird der durchschnittliche Helligkeitswert auf den Helligkeitsfaktor eingestellt. Dies erfolgt durch eine Anpassung einer Belichtungszeit der Kameramodule 14, 16 bis der aktuelle durchschnittliche Helligkeitswert und der Helligkeitsfaktor identisch sind. Liegt ein aktueller Helligkeitswert unter einem eingestellten Helligkeitsfaktor wird eine Belichtungszeit erhöht. Liegt ein aktueller Helligkeitswert über einem eingestellten Helligkeitsfaktor wird eine Belichtungszeit reduziert. Dies wird wiederholt, bis der aktuelle Helligkeitswert dem Helligkeitsfaktor entspricht. Anschließend wird in einem Schritt 80b die Beleuchtungseinheit 44 auf die Beleuchtungseinstellung des ausgewählten Datenprofils eingestellt. Dabei werden über die Steuer- und Regeleinheit 18 definiert einzelne Beleuchtungselemente 48, 50 der Beleuchtungseinheit 44 aktiviert und einzelne Beleuchtungselemente 48, 50 der Beleuchtungseinheit 44 deaktiviert. Die aktivierten Beleuchtungselemente 48, 50 werden von der Steuer- und Regeleinheit 18 auf einen auf dem ausgewählten Datenprofil hinterlegten Wert gedimmt. Über die Beleuchtungseinstellung kann gezielt eine Ausleuchtung gesteuert werden. Darauffolgend wird in einem weiteren Schritt 32b, welcher in der Figur 5 als Unterprogramm dargestellt ist, ein regulärer Filmbetrieb durchgeführt. Dabei werden von der Kameraeinheit 12 stereoskopische Bilder aufgenommen, die in Form digitaler Bilddateien an die Steuer- und Regeleinheit 18 übertragen werden. In dem Schritt 32b des regulären Betriebs werden von jeder der Kameramodule 14, 16 der Kameraeinheit 12 jeweils kurz aufeinanderfolgend zwei Bilder mit differierenden Belichtungswerten aufgenommen. Von den Belichtungswerten sind einer von einem zu dem Helligkeitsfaktor des ausgewählten Datenprofils passenden Belichtungswert und ein zweiter Belichtungswert mit einem wesentlich höher liegenden Helligkeitswert gebildet.

Anschließend werden in dem Schritt 32b die zwei Bilder jeweils eines Kameramoduls 14, 16 jeweils zu einem Bild verarbeitet. Die zwei Bilder jeweils eines Kameramoduls 14, 16 werden jeweils zu einem HDR-Bild, also einem "High Dynamic Range Image", verarbeitet. Dabei werden die beiden Bilder überlagert, wobei ein Transparenzverhältnis der Bilder jeweils abhängig von einem relativen Tonwert variiert. Dabei weisen Teilbereiche des ersten Bildes mit dem Helligkeitsfaktor, welche einen relativ hohen Tonwert aufweisen, eine geringe Transparenz auf, wobei Teilbereiche mit einem relativ niedrigen Tonwert eine hohe Transparenz aufweisen. Die Transparenzen des ersten Bildes steigen insbesondere mit sinkendem Tonwert kontinuierlich an. Dagegen weisen Teilbereiche des zweiten Bildes mit dem zweiten Belichtungswert, welche einen relativ niedrigen Tonwert aufweisen, eine geringe Transparenz auf, wobei Teilbereiche mit einem relativ hohen Tonwert eine hohe Transparenz aufweisen. Die Transparenzen des zweiten Bildes steigen insbesondere mit steigendem Tonwert kontinuierlich an. Grundsätzlich wäre jedoch auch denkbar, dass die zwei Bilder mit differierenden Belichtungswerten gleichzeitig mit differierenden Kameramodulen 14, 16 der Kameraeinheit 12 aufgenommen werden und zu einem stereoskopischen Bild verarbeitet werden.

Aus dem bearbeiteten Bildsignal wird in einem weiteren Schritt 88b von der Steuer- und Regeleinheit 18 ein Ausgangssignal mit einer stereoskopischen Bildinformation erzeugt und der Anzeigeeinheit 20 zugeführt. Die Schritte 32b und 88b werden während eines regulären Betriebs ständig widerholt, bis die Dentalbilderfassungsvorrichtung 10 deaktiviert wird.

## Patentansprüche

1. Dentalbilderfassungsvorrichtung mit einer Kameraeinheit (12) zur Aufnahme stereoskopischer Bilder, die zumindest zwei Kameramodule (14, 16) aufweist, und mit zumindest einer Steuer- und/oder Regeleinheit (18), die Signale der Kameraeinheit (12) verarbeitet und zumindest ein Ausgabesignal liefert, das dazu vorgesehen ist, einer Anzeigeeinheit (20) zugeführt zu werden, wobei die zumindest eine Steuer- und/oder Regeleinheit (18) zumindest ein Speicherelement (24) aufweist, auf welchem zumindest ein Datenprofil hinterlegt ist, und mit zumindest einer Sensoreinheit (28), welche mit der zumindest einen Steuer- und/oder Regeleinheit (18) verbunden ist, wobei die zumindest eine Steuer- und/oder Regeleinheit (18) dazu vorgesehen ist, abhängig von einem Signal der zumindest einen Sensoreinheit (28) selbsttätig ein auf dem zumindest einen Speicherelement (24) der zumindest einen Steuer- und/oder Regeleinheit (18) hinterlegtes Datenprofil auszuwählen, **dadurch gekennzeichnet, dass** auf dem zumindest einen Speicherelement (24) zumindest ein Datenprofil hinterlegt ist, welches einem Material zugeordnet ist, wobei unter einem Datenprofil ein Datensatz verstanden werden soll, welcher ein oder mehrere Einstellungen und/oder Konfigurationen umfasst, wobei der Datensatz beispielsweise einem Material und/oder einem Benutzer und/oder einer Anwendung und/oder einem Patienten zugeordnet ist und wobei unter einem Material ein Material eines zu beobachtenden Objekts verstanden werden soll, wobei die zumindest eine Steuer- und/oder Regeleinheit (18) dazu vorgesehen ist, abhängig von einem ausgewählten, auf dem Speicherelement (24) hinterlegten Datenprofil die Signale der Kameraeinheit (12) und/oder ein Ausgabesignal anzupassen, wobei auf dem zumindest einen Speicherelement (24) mehrere Datenprofile hinterlegt sind, die jeweils einem Material zugeordnet sind, wobei die Datenprofile dabei dazu ausgelegt sind, die Beleuchtungseinstellung einer Beleuchtungseinheit für eine vorteilhafte Ausleuchtung des Materials bei gleichzeitiger Vermeidung von Reflektionen anzupassen.

2. Dentalbilderfassungsvorrichtung nach Anspruch 1,
**dadurch gekennzeichnet, dass**
auf dem zumindest einen Speicherelement (24) der zumindest einen Steuer- und/oder Regeleinheit (18) mehrere zu einer Auswahl stehende Datenprofile hinterlegt sind.

3. Dentalbilderfassungsvorrichtung nach einem der vorhergehenden Ansprüche,
**gekennzeichnet durch**
zumindest eine Eingabeeinheit (26), welche zu einer Auswahl eines auf dem zumindest einen Speicherelement (24) der zumindest einen Steuer- und/oder Regeleinheit (18) hinterlegten Datenprofils vorgesehen ist.

4. Dentalbilderfassungsvorrichtung nach einem der vorhergehenden Ansprüche,
**gekennzeichnet durch**
zumindest eine Schnittstelle (90), über welche ein externes Datenprofil auf das zumindest eine Speicherelement (24) der zumindest einen Steuer- und/oder Regeleinheit (18) übertragen werden kann.

5. Dentalbilderfassungsvorrichtung nach einem der vorhergehenden Ansprüche,
**dadurch gekennzeichnet, dass**
die zumindest eine Steuer- und/oder Regeleinheit (18) dazu vorgesehen ist, eine Momentaufnahme mit einem aktuell ausgewählten Datenprofil zu verknüpfen.

## Claims

1. Dental image capturing device,
with a camera unit (12) for taking stereoscopic images, which has at least two camera modules (14, 16),
and with at least one control and/or regulation unit (18) processing the signals of the camera unit (12) and providing at least one output signal that is configured to be fed to a display unit (20),
the at least one control and/or regulation unit (18) comprising at least one memory element (24) which at least one data profile is stored on,
and with at least one sensor unit (28) which is connected to the at least one control and/or regulation unit (18),
the at least one control and/or regulation unit (18) being configured, depending on a signal of the at least one sensor unit (28), to autonomously select a data profile stored on the at least one memory element (24) of the at least one control and/or regulation unit (18),
**characterised in that** on the at least one memory element (24) at least one data profile is stored that is assigned to a material,
wherein a data profile is to mean a dataset comprising one or several settings and/or configurations,
the dataset being for example assigned to a material and/or to a user and/or to an application and/or to a patient,
and wherein a material is to mean a material of an object that is to be observed,
wherein the at least one control and/or regulation unit (18) is configured to adapt the signals of the camera unit (12) and/or an output signal depending on a selected data profile that is stored on the memory element (24),
wherein on the at least one memory element (24) several data profiles are stored which are respectively assigned to a material,
the data profiles being designed, for an advantageous illumination of the material, to adapt the illumination setting of an illumination unit, at the same time avoiding reflections.

2. Dental image capturing device according to claim 1,
**characterised in that**
several data profiles provided for selection are stored on the at least one memory element (24) of the at least one control and/or regulation unit (18).

3. Dental image capturing device according to one of the preceding claims,
**characterised by**
at least one input unit (26), which is configured for a selection of a data profile stored on the at least one memory element (24) of the at least one control and/or regulation unit (18).

4. Dental image capturing device according to one of the preceding claims,
**characterised by**
at least one interface (90), via which an external data profile is transferable to the at least one memory element (24) of the at least one control and/or regulation unit (18).

5. Dental image capturing device according to one of the preceding claims,
**characterised in that**
the at least one control and/or regulation unit (18) is configured to link a snapshot to a currently selected data profile.

## Revendications

1. Dispositif d'enregistrement d'image dentaire,
avec une unité caméra (12) pour la prise des images stéréoscopiques, comprenant au moins deux modules de caméra (14, 16),
et avec au moins une unité de commande et/ou régulation (18) qui traite les signaux de l'unité caméra (12) et produit au moins un signal de sortie prévu à être alimenter à une unité d'affichage (20),
l'au moins une unité de commande et/ou régulation (18) comprenant au moins un élément de stockage (24), sur lequel au moins un profil de données est stocké,
et avec au moins une unité de captage (28) qui est raccordée à l'au moins une unité de commande et/ou régulation (18),
l'au moins une unité de commande et/ou régulation (18) étant configurée, en dépendance à un signal de l'au moins une unité de captage (28), à sélectionner en autonomie un profil de données stocké sur l'au moins un élément de stockage (24) de l'au moins une unité de commande et/ou régulation (18), **caractérisé en ce que** sur l'au moins un élément de stockage (24) au moins un profil de données est stocké qui est alloué à un matériau,
un profil de données étant à signifier un jeu de données comprenant un réglage ou plusieurs réglages et/ou une configuration et/ou plusieurs configurations,
le jeu de donnée étant alloué par exemple à un matériau et/ou à un utilisateur et/ou à une application et/ou à un patient et
un matériau étant à signifier un matériau d'un objet qui est à observer,
où l'au moins une unité de commande et/ou régulation (18) est configurée à adapter les signaux de l'unité caméra (12) et/ou un signal de sortie en dépendance à un profil de données sélectionné, qui est stocké sur l'élément de stockage (24),
où plusieurs profils de données, chacun desquels étant alloué à un matériau respectif, sont stockés sur l'au moins un élément de stockage (24),
où pour une illumination avantageuse du matériau, les profils de donnée sont configurés à adapter le réglage d'illumination d'une unité d'illumination en simultanément évitant des réflexions.

2. Dispositif d'enregistrement d'image dentaire selon la revendication 1,
**caractérisé en ce que**
plusieurs profils de données pourvus pour la sélection sont stockés sur l'au moins un élément de stockage (24) de l'au moins une unité de commande et/ou régulation (18).

3. Dispositif d'enregistrement d'image dentaire selon l'une des revendications précédentes,
**caractérisé par**
au moins une unité d'entrée (26) configurée à sélectionner un profil de données stocké sur l'au moins un élément de stockage (24) de l'au moins une unité de commande et/ou régulation (18).

4. Dispositif d'enregistrement d'image dentaire selon l'une des revendications précédentes,
**caractérisé par**
au moins une interface (90) par le biais de laquelle un profil de données externe peut être transféré sur l'au moins un élément de stockage (24) de l'au moins une unité de commande et/ou régulation (18).

5. Dispositif d'enregistrement d'image dentaire selon l'une des revendications précédentes,
**caractérisé en ce que**
l'au moins une unité de commande et/ou régulation (18) est configurée à relier un instantané avec un profil de données actuellement sélectionné.
